# EUROPEAN PATENT APPLICATION

(11) **EP 4 360 619 A1**
(43) Date of publication of application: **01.05.2024**
(21) Application number: 22306613.5
(22) Date of filing: 25.10.2022
(51) Int. Cl.: A61K 9/00, A61K 31/165, A61K 31/445, A61K 47/34, A61P 29/00, A61P 19/02, A61K 9/10, A61K 9/16

(54) **DOSAGE FORM FOR INTRA-ARTICULAR INJECTION COMPRISING COLCHICINE AND AN ANESTHESIC AGENT IN THE TREATMENT OF CRYSTAL-AND NON-CRYSTAL ASSOCIATED ACUTE INFLAMMATORY ARTHRITIS**

(71) Applicant: PK MED, 75013 Paris (FR)
(72) Inventor: The designation of the inventor has not yet been filed
(74) Representative: Cabinet Nony

(57) **Abstract**

The present invention relates to a pharmaceutical composition suitable for an intra-articular injection comprising an anesthetic agent and a controlled release dosage form comprising colchicine. It further relates to a pharmaceutical composition under the form of a powder comprising an anesthetic agent and controlled release dosage form comprising colchicine, wherein said controlled release dosage form comprising colchicine are under the form of microparticles. It further relates to a pharmaceutical composition under the form of a powder comprising an anesthetic agent and controlled release dosage form comprising colchicine, wherein said controlled release dosage form comprising colchicine are under the form of microparticles, to a kit and to the use of said pharmaceutical compositions or kit in the treatment of crystal-associated and non-crystal associated acute inflammatory arthritis, in particular a crystal-associated arthropathy or flares or acute forms of tendinitis and capsulitis by intra-articular injection into a joint of said pharmaceutical composition, and in providing pain relief immediately and all over the treatment.

## Description

The present invention relates to the field of the treatment of inflammatory pain in joints by colchicine in combination with an anesthetic agent. Inflammatory pain in joints aimed at in the present invention may be crystal-associated and non-crystal associated acute arthritis, in particular arthropathies attacks or flares, and acute forms of tendinitis and capsulitis and merely targets acute diseases. Colchicine and an anesthetic agent are administered locally by intra articular injection and colchicine is administered *via* a controlled release dosage form.

### BACKGROUND

Inflammatory arthritis are a group of chronic auto-immune and auto-inflammatory diseases characterized by joint inflammation with symptoms such as pain, swelling, stiffness and decreased range of motion. Patients generally experience alternating acute periods with highly intense symptoms in one or several joints and periods of inactivity. Inflammatory arthritis includes several diseases and conditions such as Rheumatoid arthritis, Ankylosing spondylitis, Juvenile arthritis, Psoriatic arthritis, Lupus or Microcrystalline diseases.

In particular, the class of microcrystalline arthropathies corresponds to an inflammation of local tissue caused by the deposition of microcrystals in joints. These crystals trigger an inflammatory reaction and severe joints pain. This class of diseases includes several conditions which differ in particular in the nature of the crystals: Gout (monosodium urate - MSU), chondrocalcinosis (calcium pyrophosphate - CPP), calcifying tendinitis (calcium phosphate) and Milwaukee shoulder syndrome (calcium phosphate). In this context, the crystal-associated arthropathies are characterized by multiple, extremely painful acute attacks or flares followed by pain, disability and impaired quality of life.

Tendinitis is an inflammation of a tendon that can be secondary to several factors including traumatic, degenerative, iatrogenic i.e. drug-induced, age and inflammation, responsible of pain and disability. Tendinitis can occur at any age, but it is more common among adults who do a lot of sport or any intense cyclic tractions on the tendon. Older people are also susceptible, because the tendons tend to lose elasticity and become weaker with age. A chronic pain presentation also exists in calcifying tendinitis, as well as an acute form of calcifying tendinitis.

Capsulitis is an inflammation of a capsule generating pain and stiffness. Capsulitis frequently affects the shoulder and, in this case, may also be called frozen shoulder.

For tendinitis and capsulitis, the present invention mainly targets their acute form and for example calcifying tendinitis.

Treatment recommendations for these acute joint diseases are most often symptomatic and aim to control the pain. Generally, the first line options are mainly oral nonsteroidal anti-inflammatory drugs (NSAIDs), oral or intra-articular corticosteroids or oral colchicine (reglementary approved for gout). When it is possible and when the joints are accessible, i.e. in the case of large joints (knees, ankles, wrists, elbows and shoulders), intra-articular corticosteroid injection is considered by practitioners to avoid systemic administration and thus avoid the risk of significant side effects.

Intra-articular administration of corticosteroid, used for several decades in joint diseases in general, permits an improvement in pain and mobility, with non-lasting effects (a few weeks in the case of an immediate release form). However, the toxicological aspect of these administrations is quite problematic and controversial. Indeed, systemic effects are mentioned for weeks following the injection including Cushing's syndrome, hyperglycemia, decreased bone density or a risk of infections. The latter being the consequence of the direct introduction of an infectious agent into the articulation or by reducing the immune response and allowing a latent tuberculosis infection to be reactivated. This type of treatment is in particular not adapted for patients at risk, i.e. for example with comorbidities, like diabetes or osteoporosis. In addition, deleterious effects are also described on local tissue as damage to articular cartilage and tendons.

Consequently, in medical practice, the frequency and dose of intra-articular corticosteroid injections are limited. For the immediate release galenic forms it is therefore recommended a maximum of three injections per year/per site and a minimum interval of three months between two injections. A first intra-articular sustained-release formulation of corticosteroid (Zilretta^{®} by Flexion Therapeutics) was approved in 2017 by the FDA for osteoarthritis, allowing continuous treatment for months. However, this product is not approved for repeated administrations.

Intra-articular administration of corticosteroids therefore does not seem to be an optimal treatment for extremely painful attacks, such as gout attack or chondrocalcinosis.

Colchicine is a drug characterized by pleiotropic effects related to its capacity to bind to tubulins and disturb microtubule polymerization, destabilizing the cytoskeleton and all the cellular processes, including cell division, migration and cell shape. Among these effects, colchicine affects the immune system and downregulates multiple inflammatory pathways, leading to a decrease in neutrophil function and migration. Colchicine is therefore used and approved in several inflammatory diseases such as Familial Mediterranean Fever, Behçet disease and Microcrystalline diseases.

Colchicine has indeed been used for a very long time for the non-specific treatment of crystal-associated arthropathies. In the past 50 years, the use of short courses of fast-acting NSAIDs and corticoids have been considered with colchicine as the oral drugs of choice for the symptomatic treatment of acute gout and pseudogout. In the article George Nuki, MB, FRCP "Colchicine: its Mechanism of Action and Efficacy in Crystal-Induced Inflammation", Current Rheumatology Reports, 2008, 10:218-227, prophylactic and therapeutic efficacy of oral administration of colchicine is reviewed. Still in said article, side effects of colchicine are extensively described, such as powerful purgative effects, bone marrow suppression, neuromyopathy and rhabdomyolysis, especially in patients with renal insufficiency as well as high mortality associated with overdosage.

Colchicine has thus an exceptionally narrow therapeutic index. Therefore, for all these reasons, the use of colchicine remains limited although its efficacy has been largely proven.

Moreover, although it is known to treat inflammation in joints, i.e. at site, by directly administering an active substance therein, it has been found that the active substance escapes relatively easily from said joint, thus reducing the therapeutic effect.

Colchicine encapsulation for sustained release has been reported in different carriers, such as in polymeric microspheres (Das, G. S. et al. "Colchicine Encapsulation within Poly(Ethylene Glycol)-Coated Poly(Lactic Acid)/Poly(ε-Caprolactone) Microspheres-Controlled Release Studies" Drug Delivery, 7: 7544, 129-138 (2000)). In this article, colchicine has been investigated in the treatment of other diseases, such as restenosis after angioplasty or vascular injury, and on this occasion, attempts of encapsulation of colchicine within poly(lactic acid)/poly(ε-Caprolactone) microspheres were performed for local delivery of colchicine. Another composition comprising biodegradable microparticles containing colchicine was further described seeking the treatment of the same pathology, i.e. restenosis, through intramural delivery, as reported in Gradus-Pizlo Irmina et al: "local delivery of biodegradable microcapsules containing colchicine or a colchicine analogue: effects on restenosis and implications of catheter-based drug delivery", Journal of The American College of Cardiology, vol.26, no. 6, pages 1549-1557. It clearly comes out that the therapeutic target is completely different from the one of the present invention. In addition, the results are not encouraging and point out toxicity risks. Indeed, signs of local toxicity were observed in the muscle layers overlying and fed by arteries infused with the microparticles containing colchicine or colchicine analogue but not controlled microparticles.

In addition, colchicine has previously been reported in US5,747,060, again for other effects than the one as directed to in the framework of the present invention, as acting on the prolongation of the duration of the anesthetic effect produced by the local administration of an anesthetic. Colchicine is thus co-administered in example 1 of said document around the sciatic nerve with bupivacaine, under an immediate release form, i.e. directly added into an aqueous solution. Intra-articular injection is not described in said document nor the activity of colchicine in the treatment of inflammatory pain in joints.

WO2006/066419 discloses the same effect of colchicine with respect to the prolongation of its anesthetic effect and namely a combination of a vanilloid receptor agonist and another molecule, possibly colchicine. An example discloses a composition comprising a mixture of resiniferatoxin and colchicine injected intra-articularly for the treatment of capsulitis. Said injection is however additionally dedicated for an immediate release not seeking to control the local profile and systemic pharmacokinetics of colchicine, nor to extend its therapeutic exposure.

The use of colchicine for the treatment of crystal-associated arthropathies is well established. However, nowadays, colchicine is only commercialized under an immediate release oral dosage form, classically with a daily dosage for the treatment of the crystal-associated arthropathy around 1 mg per day. The FDA-approved dosage for the treatment of acute gout flare is 1.2 mg colchicine at the first sign of the flare followed by 0.6 mg one hour late (https://www.fda.gov/drugs/postmarket-drug-safety-information-patients-and- providers/colchicine-marketed-colcrys-information). The recommended colchicine first-line treatment for acute flare by EULAR is a loading dose of 1 mg followed 1 hour later by 0.5 mg (Richette, P. et al. 2016 updated EULAR evidence-based recommendations for the management of gout. Ann. Rheum. Dis. 76, 29-42 (2017)). However, as explained above, and illustrated herein after in more details, reaching colchicine therapeutically active dose locally is hindered by the low tolerance or high toxicity of orally administered colchicine rendering the drug prescription extremely complicated.

Colchicine was recently described in a nanoemulsion system injected into the joint of Swiss albino mice model with MSU (Monosodium Urate) crystal-induced gouty arthritis: Aboumanei et al, "intra-articular formulation of colchicine loaded nanoemulsion systems for enhanced locoregional drug delivery: In vitro characterization, 99mTc coupling and in vivo biodistribution studies", DOI: 10.1080/03639045.2021.1934865. The biodistribution pattern of ^{99m}Tc-ColNE-5 as well as ^{99m}Tc-Col solution (^{99m}TcColS) was studied. However, said disclosure is limited to an observation of a radiolabeled colchicine which is thus a different molecule from colchicine itself and within a period not exceeding 24 hours. It is well known that labelling with radionuclide like ^{99m}Tc can have a strong impact on the pattern of biodistribution of the labelled entity due to modification of its charge, lipophilicity and stability (Decristoforo et al. "The influence of chelator on the pharmacokinetics of 99mTc-labelled peptides." Q. J. NUCL. MED. 46 3 (2002): 195-205). Moreover, said article is silent on the technical problem of narrow therapeutic index as explained above and very specific to the colchicine, i.e. the maximal systemic concentration to be avoided for toxicity reasons. Said article is thus also silent on microparticulate systems with targeted dosages able to reach effective local concentration for combatting the acute forms of the considered pathologies, with no toxicity at a systemic level. It namely appears in figure 3 of said article that ColNE-5, i.e. « colchicine nanoemulsion system » provides a release of 40% of colchicine in less than 3 hours, which is contrary to the fulfilment of a controlled systemic concentration of colchicine for avoiding side effects, as required in the present invention. Achieving release profiles not having such a quick release just after the administration, also called "burst release", as explained herein after, that can be detrimental in terms of side effects in such an extent, implies as such a non-obvious approach which was not predicable from said article. In other words, no colchicine controlled release profile suitable for human healing, and even less in combination with an anesthetic agent, is revealed in said article, and even more particularly for the treatment of a crystal-associated or non-crystal associated acute inflammatory arthritis, in particular effective in treating non-crystal associated acute arthritis, such as Rheumatoid arthritis, Ankylosing spondylitis, Juvenile arthritis, Psoriatic arthritis, Lupus or in treating crystal-associated arthropathies attacks or flares, such as Gout, Chondrocalcinosis, calcifying tendinitis and Milwaukee shoulder syndrome.

IV injection of colchicine is considered effective in the treatment of acute gout attack with a dose not exceeding 2-3 mg per single injection (Nuki, G. Colchicine: Its mechanism of action and efficacy in crystal-induced inflammation. Curr. Rheumatol. Rep. 10, 218-227 (2008)). However even if GI side effects can be avoided using IV administration, severe toxicity was associated with inappropriate dosing (Wallace SL, S. J. Review: systemic toxicity associated with the intravenous administration of colchicine-guidelines for use. J Rheumatol. 15, 495-9 (1988), Bonnel, R. A., Villalba, M. L., Karwoski, C. B. & Beitz, J. Deaths associated with inappropriate intravenous colchicine administration. J. Emerg. Med. 22, 385-387 (2002)). This led authorities to eliminate the use of IV colchicine for acute gout treatment (Zhang, W. et al. EULAR evidence-based recommendations for gout. Part II: Management. Report of a task force of the EULAR Standing Committee for International Clinical Studies Including Therapeutics (ESCISIT). Ann. Rheum. Dis. 65, 1312-1324 (2006)). Attempts of i.v. injection of colchicine have furthermore largely discouraged the man skilled in the art to envision injection of colchicine, as reported in E.Niel et al, "Colchicine today", Joint Bone Spine 73 (2006) 672-678.

Therefore, it remains a need to develop novel dosage forms, route of administrations and targeted dosage for colchicine, both for obviating the side-effects, in particular observed during systemic administration, and improving the efficacy by optimizing the available dose of colchicine, in particular for extending its local duration of action at the painful site, in particular by increasing the local concentration, in particular for the treatment of acute crisis of crystal-associated inflammatory arthritis and non-crystal associated inflammatory arthritis while providing pain relief and inflammation decrease immediately and throughout the duration of the flare.

The invention has for purpose to meet the needs, thereby both obtaining increased advantage from its action against the inflammation and pain relief in the treatment of crystal-associated and non-crystal associated acute inflammatory arthritis and restricting the level of any side effects. This means that colchicine aims at acting on the inflammation and/or pain component of the crystal-associated and non-crystal associated acute inflammatory arthritis.

One of the advantages of the present invention is to provide a new mean to achieve a particular colchicine release profile which respects an adequate ratio between the local or synovial concentration of colchicine and the systemic concentration thereof, suitable for offering the best therapeutic action locally and at the same time avoiding potential systemic toxicity while providing pain relief and inflammation decrease immediately and throughout the duration of the flare. The coadministration of an anesthetic agent indeed allows to quickly relieve pain, with an action that can last up to 72 hours.

Due to the reduced side-effects obtained by the administration of the pharmaceutical composition according to the present invention, it is furthermore particularly suitable for people suffering from chronic comorbidities (Hypertension, Chronic Kidney diseases, Diabete, Heart diseases, Immunosuppression...) fairly prevalent conditions in microcrystalline joint diseases (50% of Gout patients having at least 3 comorbidities). Indeed, the management of microcrystalline joint diseases patients having multiple comorbidities, remains a challenge for medical practitioners due to frequent and major contraindications to either Colchicine, NSAIDS or Corticosteroids. In comparison to the existing oral colchicine treatment, the present invention, in addition to avoid gastrointestinal side effects, prevents major toxicity risks for patients suffering from renal or hepatic impairments or for co-medicated patients with CYP3A4/PGP drug inhibitors, which are fairly prevalent medications.

### SUMMARY OF THE INVENTION

According to a first aspect, herein is provided a pharmaceutical composition suitable for an intra-articular injection comprising an anesthetic agent and a controlled release dosage form comprising colchicine.

According to a second main embodiment, herein is further provided a pharmaceutical composition under the form of a powder comprising an anesthetic agent and controlled release dosage form comprising colchicine, wherein said controlled release dosage form comprising colchicine are under the form of microparticles, in particular microparticles comprising a polymeric matrix, more particularly having a mean particle size equal or greater than 10 µm, and even more particularly as defined herein after, said anesthetic agent being in particular as defined herein after.

According to a third main embodiment, herein is further provided a kit or article of manufacture comprising, in separate compartments (i) an aqueous injection vehicle, optionally comprising an immediate release form of an anesthetic agent, identical or different from the anesthetic agent present in (ii) as defined herein after and (ii) a controlled release dosage form comprising colchicine, in particular as defined herein after or powder as defined herein after and an anesthetic agent, in particular as defined herein after, wherein the kit or article of manufacture optionally comprises an excipient selected from a group consisting of a tonicity-enhancing agent, a wetting-agent, a viscosity-enhancing agent, a density-enhancing agent or a mixture thereof, for preparing a pharmaceutical composition suitable for an intra-articular injection.

Herein is further provided a pharmaceutical composition as defined above and as detailed herein after or as obtained by mixing the two compartments of a kit as defined above and as detailed herein after, for use in the treatment of crystal-associated and non-crystal associated acute inflammatory arthritis, in particular a crystal-associated arthropathy or flares or acute forms of tendinitis and capsulitis by intra-articular injection into a joint of said pharmaceutical composition, in particular providing pain relief and inflammation decrease, and more particularly immediately and all over the flare duration, wherein the time required to release 80% by weight of the colchicine is greater than 1 day and may reach 15 days, wherein the colchicine is present in a concentration ranging from 2.5 to 2500 µg per ml of the sterile and injectable dosage form, in particular a suspension, wherein the anesthetic agent is present in a concentration ranging from 0.05 to 120 mg per ml of the sterile and injectable dosage form, in particular a suspension, wherein 80% of the anesthetic agent is released in maximum 3 days and wherein the pharmaceutical composition has a volume ranging from 0.1 ml to 5 ml.

The pharmaceutical composition suitable for an intra-articular injection as described herein after, in particular under the form of a suspension, is effective in treating crystal-associated and non-crystal associated acute inflammatory arthritis, in particular effective in treating non-crystal associated acute arthritis, such as Rheumatoid arthritis, Ankylosing spondylitis, Juvenile arthritis, Psoriatic arthritis, Lupus or in treating crystal-associated arthropathies attacks or flares, such as gout, chondrocalcinosis, calcifying tendinitis and Milwaukee shoulder syndrome with minimal long-term side effects.

Said pharmaceutical composition suitable for an intra-articular injection is suitable for local administration by injection into a site at or near the site of pain.

### DETAILED DESCRIPTION OF THE INVENTION

As apparent from the BACKGROUND paragraph above, the prescription of colchicine is nowadays delicate as only oral compositions are available and high risk of toxicity have been observed, such as gastrointestinal symptoms and neuromuscular involvement. Risks linked to interaction with other active agents have further been reported. And again, toxicity constraints frankly have discouraged the local injection for decades.

Indeed, it has been established in the literature that effective local dose of 0.015 mg/kg to 0.030 mg/kg colchicine is recommended, that at 0.1 mg/kg the toxicity limit is reached, and that at 0.8 mg/kg lethal dose is reached (E.Niel et al, "Colchicine today", Joint Bone Spine 73 (2006) 672-678). It is thus concluded that the narrow therapeutic margin for colchicine is a source of concern for prescribing physicians.

The inventors have found that compared to the treatment of oral colchicine or intra-articular corticosteroids in joint pain diseases, the intra-articular injection of a pharmaceutical composition according to the present invention, in particular under the form of a suspension, for a controlled release of colchicine has at least, equivalent, and even higher efficiency. It provides better pain relief as soon as administered. In this invention, the time required to release 80% by weight of the colchicine, in particular from the controlled release dosage form comprising it, and more particularly from the microparticles, is greater than 1 day and may reach 15 days, when the colchicine is present in a concentration ranging from 2.5 to 2500 µg per ml of the sterile and injectable dosage form, in particular a suspension, and the time required to release 80% of the anesthetic agent, in particular ropivacaine, is less than 3 days and it is present in a concentration ranging from 0.05 to 120 mg per ml of the sterile and injectable dosage form, in particular a suspension.

The diseases for which the use of said pharmaceutical composition is dedicated are very intense. Thus, having treatments perfectly adapted for achieving the most appropriate dosage is crucial.

The use or method of treatment according to the present invention also has the advantage of reducing the significant toxicity risks, linked to drug interaction with colchicine when administered *via* a systemic route, and in particular in the elderly, or patients with kidney or liver failure as explained herein after.

In comparison to intra-articular corticosteroids, this treatment also has the advantage to avoid systemic effects such as Cushing's syndrome, hyperglycemia, hypertension, decreased bone density or even the risk of infections. In addition, it decreases the risk of deleterious effects on local tissue as damage to articular cartilage and tendons.

### Definitions

As used herein, the term "patient" refers to either an animal, such as a valuable animal for breeding, company or preservation purposes, or preferably a human or a human child, which is afflicted with, or has the potential to be afflicted with one or more diseases and conditions described herein.

In particular, as used in the present application, the term "patient" refers to a mammal, including a non-human mammal such as a rodent, cat, dog, or primate, or a human; preferably said subject is a human and also extends to birds.

The identification of those patients who are in need of treatment of herein-described diseases and conditions is well within the ability and knowledge of one skilled in the art. A veterinarian or a physician skilled in the art can readily identify, by the use of clinical tests, physical examination, medical/family history or biological and diagnostic tests, those patients who are in need of such treatment.

In the context of the invention, the term "treating" or "treatment", as used herein, means reversing, alleviating, inhibiting the progress of non-crystal associated acute inflammatory arthritis, such as Rheumatoid arthritis, Ankylosing spondylitis, Juvenile arthritis, Psoriatic arthritis, Lupus or in treating crystal-associated acute inflammatory arthritis, in particular crystal-associated arthropathies attacks or flares, such as gout, chondrocalcinosis, calcifying tendinitis and Milwaukee shoulder syndrome, and acute forms of tendinitis and capsulitis.

As used herein, an "effective amount" refers to an amount of a compound of the present invention which is effective in reducing, eliminating, treating or controlling the symptoms of the herein-described diseases and conditions, *i.e.* crystal-associated and non-crystal associated acute inflammatory arthritis, in particular non-crystal associated acute arthritis, such as Rheumatoid arthritis, Ankylosing spondylitis, Juvenile arthritis, Psoriatic arthritis, Lupus or in treating and crystal-associated arthropathies attacks or flares, such as gout, chondrocalcinosis, calcifying tendinitis and Milwaukee shoulder syndrome, and acute forms of tendinitis and capsulitis. The term "controlling" is intended to refer to all processes wherein there may be a slowing, interrupting, arresting, or stopping of the progression of the diseases and conditions described herein, but does not necessarily indicate a total elimination of all diseases and condition symptoms.

The term "treatment-effective amount" refers to a concentration of compound that is effective in treating crystal-associated and non-crystal associated acute inflammatory arthritis, in particular non-crystal associated acute arthritis, such as Rheumatoid arthritis, Ankylosing spondylitis, Juvenile arthritis, Psoriatic arthritis, Lupus or in treating crystal-associated arthropathies attacks or flares, *e.g.* leads to a reduction in inflammatory pain, following examination when administered after initiation of the inflammatory pain has occurred.

As used herein, the term "pharmaceutically acceptable" refers to those compounds, materials, excipients, compositions, or dosage forms which are, within the scope of sound medical judgment, suitable for contact with the tissues of human beings and animals without excessive toxicity, irritation, allergic response or other problem complications commensurate with a reasonable benefit/risk ratio.

The term "pharmaceutically acceptable carrier, adjuvant, or vehicle" may refer to any pharmaceutically acceptable excipient, such as a non-toxic carrier, adjuvant, or vehicle that does not destroy the pharmacological activity of the compound with which it is formulated.

Within the framework of the present invention, the term "painful site" or "site of the pain" means the joint area causing pain where the product can be injected. It can be the knee joint, the hip joint, metacarpophalangeal joints, the shoulder joint, the wrist joint, the elbow joint, the metatarsophalangeal joint, the ankle joint, the vertebral joint and the midfoot joints.

The terms "controlled release composition" or "controlled release microparticle" mean the composition or the microparticle allow a release into the body of a specified amount over a specified period of time of an active ingredient, namely a specific pharmacokinetic profile. The term "controlled release" encompasses all the type of releases that are modified in comparison to an immediate release. In other words, the term "controlled release" is equivalent to "modified release" and encloses extended releases as defined herein after as well as delayed release and pulse releases.

The terms "extended release", "prolonged release" and "sustained release" are considered equivalent in the framework of the present invention. This type of release means that the release is prolonged over time in comparison to an immediate release, i.e. it means that the active ingredient is released slowly over time, allowing a less frequent intake of the drug for the patient. In other words, the active ingredient is progressively released over a specific period of time, generally aiming at less side effects as well by decreasing the maximal concentration.

The term "drug loading content" means the mass ratio of the drug in the microparticles to the mass of microparticles.

The term "mean particle size" or D50 means the particle diameter in microns that splits the particle volume distribution with half above and half below this diameter.

The term "D10=x µm" as mainly used in the example means that 10% of the particles have a size of x µm or less.

The term "D90=y µm" as mainly used in the example means that 90% of the particles have a size of y µm or less.A controlled or modified release may of course result from the combination of an immediate release and a controlled release.

### COLCHICINE

Colchicine is an alkaloid extracted from the corm of the meadow saffron or autumn crocus (*Colchicum autumnale*). Its IUPAC name is N-[(7S)-1,2,3,10-tetramethoxy-9-oxo-6,7-dihydro-5H-benzo[a]heptalen-7-yl]acetamide.(CAS 64-86-8)

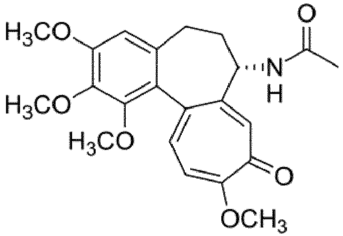

Colchicine may exist in racemic of enantiomeric form. All said forms are encompassed within the scope of the invention.

Moreover, it may exist in various crystalline forms depending on the solvent used for obtaining them. Chloroform, dichloromethane, ethyl acetate, benzene or any other suitable solvent may be cited among possible solvents. All such crystalline forms also form part of the present invention.

It is mainly known as gout suppressant and as an anti-inflammatory agent *via* its tubulin interaction activity. It is administered by the oral route although intravenous administration was also attempted and then abandoned for excessive toxicity reasons.

### ANESTHETIC AGENT

The anesthetic agent may be selected from lidocaine, ropivacaine, bupivacaine, levobupivacaine, capsaicin, mepivacaine, prilocaine, pharmaceutically acceptable salts thereof and mixtures thereof.

In one embodiment, the anesthetic agent is selected from lidocaine, bupivacaine, ropivacaine and mepivacaine, pharmaceutically acceptable salts thereof and mixtures thereof.

In one embodiment, the anesthetic agent is selected from lidocaine, ropivacaine, bupivacaine, levobupivacaine, capsaicin, mepivacaine, prilocaine, pharmaceutically acceptable salts thereof and mixtures thereof, in particular from lidocaine, bupivacaine, ropivacaine and mepivacaine, and more particularly ropivacaine.

The anesthetic agent may be partially or totally under an immediate release form or is partially or totally under a controlled release form, said controlled release form being distinct from the controlled release dosage form comprising colchicine or totally of partially being comprised in it, such as under the form of microparticles comprising a polymeric matrix, in particular as defined herein after with respect to the colchicine microparticles, under the form of a dehydrated hydrogel, under the form of multivesicular liposomes or under the form of an in-situ forming depot.

In other words, the anesthetic agent may be formulated so as to achieve an immediate release or a sustained release or a mixture of immediate release and sustained release.

The presence of said anesthetic agent affords an immediate pain relief just after the intra-articular injection of the pharmaceutical composition. The comfort of the patient may thus be improved. When under a sustained release form, said relief of pain may be prolonged. In one embodiment, said anesthetic effect may be prolonged 1 day or 2 days or 3 days, in particular prolonged 1 day.

Various embodiments of formulations are detained herein after in the paragraph "PHARMACEUTICAL COMPOSITIONS AND KITS".

### THERAPEUTIC USE AND METHODS

In one embodiment, the pharmaceutical composition suitable for an intra-articular injection as described herein after delivers colchicine in a dose and in a controlled release manner such that the concentration level of colchicine at the treated articular site is above the systemic dosage classically obtained after an oral administration, i.e. above 5 nM (2 ng/mL), and the systemic concentration level stays below the value of 15 nM (6 ng/mL). (Terkeltaub, R. A. et al. High versus low dosing of oral colchicine for early acute gout flare: Twenty-four-hour outcome of the first multicenter, randomized, double-blind, placebo-controlled, parallel-group, dose-comparison colchicine study. Arthritis Rheum. 62, 1060-1068 (2010)).

Herein is thus provided a pharmaceutical composition according to the present invention and as much more detailed herein after or as obtained by mixing the two compartments of a kit as much more detailed herein after, for use in the treatment of crystal-associated and non-crystal associated acute inflammatory arthritis, in particular a crystal-associated arthropathy or flares or acute forms of tendinitis and capsulitis by intra-articular injection into a joint of said pharmaceutical composition, in particular providing pain relief and inflammation decrease, and more particularly immediately and all over the flare duration, wherein the time required to release 80% by weight of the colchicine is greater than 1 day and may reach 15 days, wherein the colchicine is present in a concentration ranging from 2.5 to 2500 µg per ml of the sterile and injectable dosage form, in particular a suspension, wherein the anesthetic agent is present in a concentration ranging from 0.05 to 120 mg per ml of the sterile and injectable dosage form, in particular a suspension, wherein 80% of the anesthetic agent is released in maximum 3 days and wherein the pharmaceutical composition has a volume ranging from 0.1 ml to 5 ml.

According to one embodiment, said pharmaceutical composition for use is further characterized in that the crystal-associated and non-crystal associated acute inflammatory arthritis is selected from non-crystal associated acute arthritis, such as Rheumatoid arthritis, Ankylosing spondylitis, Juvenile arthritis, Psoriatic arthritis, Lupus and crystal-associated arthropathy attacks or flares or acute forms of tendinitis and capsulitis, and more particularly selected from gout, chondrocalcinosis, calcifying tendinitis and Milwaukee shoulder syndrome.

According to another embodiment, the pharmaceutical composition of the invention is further characterized in that it is effective to maintain systemic concentration of colchicine after 12 hours lower than 5 ng/ml, particularly lower than 2 ng/ml and more particularly lower than 1 ng/ml and to maintain synovial concentration of colchicine greater than 1 ng/ml, in particular comprised between 1 and 500 ng/ml, more particularly comprised between 1 and 250 ng/ml, more particularly comprised between 1 and 200 ng/ml, for example between 1 and 100 ng/ml, and even more particularly between 1 and 50 ng/ml.

According to another embodiment, the pharmaceutical composition of the invention is further characterized in that it is effective to maintain synovial concentration of anesthetic agent, in particular ropivacaine, comprised between 0.05 and 50 mg/ml, in particular between 0.05 and 25 mg/ml, and more particularly between 0.05 to 10 mg/ml, for example between 0.05 and 3 mg/ml, said synovial concentration being able to last 1 hour, 4 hours, 8 hours, 1 day, 1.5 days or 2 days or 3 days.

The particular release profile allowing a tolerable systemic concentration of colchicine and at the same time a local concentration of colchicine suitable for having an appropriate therapeutic effect, which represents the very core of the invention, has never been obtained before. It comes out that through a particular ratio between the local or synovial concentration of colchicine and systemic concentration of colchicine obtained with the pharmaceutical composition according to the invention, i.e. with a particular *in vitro* dissolution rate and/or *in vivo* release rate, the inventors have discovered a surprisingly well adapted mean for treating the patients while avoiding side effects.

According to one particular embodiment, the ratio between the local or synovial concentration of colchicine and systemic concentration of colchicine obtained after injection into a joint of a pharmaceutical composition according to the present invention may range between 100 and 2000 in human as described/mentioned in 1) Petit, A., Redout, E. M., van de Lest, C. H., de Grauw, J. C., Müller, B., Meyboom, R., van Midwoud, P., Vermonden, T., Hennink, W. E., & René van Weeren, P. (2015). Sustained intra-articular release of celecoxib from in situ forming gels made of acetyl-capped PCLA-PEG-PCLA triblock copolymers in horses. Biomaterials, 53, 426-436. https://doi.org/10.1016/j.biomaterials.2015.02.109 and 2) Kraus VB, Conaghan PG, Aazami HA, Mehra P, Kivitz AJ, Lufkin J, Hauben J, Johnson JR, Bodick N. Synovial and systemic pharmacokinetics (PK) of triamcinolone acetonide (TA) following intra-articular (IA) injection of an extended-release microsphere-based

For determining said local and systemic concentration of colchicine, the man skilled in the art may use any known method. For example, a LC-MS analysis may be used.

Illustration of said particular control of the release of colchicine as achieved in the framework of the present invention may also be illustrated *via in vitro* dissolution profiles as performed in examples 1 to 3. Illustration of said particular control of the release of the anesthetic agent, in particular ropivacaine, as achieved in the framework of the present invention may also be illustrated *via in vitro* dissolution profiles as performed in example 4.

According to one embodiment, the dissolution medium that may be used for performing such dissolution test may be a phosphate buffered saline 10 mM (pH 7.4).

According to one embodiment, the rotation speed may range from 50 to 200, in particular from 60 to 100 rpm. This agitation may be obtained thanks to shaking incubator, in particular with horizontal motion.

According to one embodiment, the dissolution of colchicine is measured for example by UV analysis using a UV spectrophotometer, for example at 350 nm, at different intervals, for example at 1 day, 2 days, 3 days, 5 days, 7 days, 8 days, 9 days, 10 days and 13 days.

By way of example, a dissolution test for colchicine microparticles may be performed according to the herein after detailed method:
Aliquots of 20 mg microparticles are suspended in 50 mL of phosphate buffered saline (10 mM, pH 7.4) containing 0.02% sodium azide maintained at 37°C in a horizontal shaking. At different intervals, 3 mL of the media are removed and centrifuged at 4000 rpm for 3 minutes. The supernatant is analyzed by UV at 350 nm. Right after UV analysis, the sample is put back in the dissolution flask.

As above mentioned, the controlled release form is administered locally to treat pain and inflammation in the joints. Local administration of the formulation may occur by injection into the intra-articular space or peri-articular space at or near the site of a patient's pain, including the metatarsophalangeal joints, the metacarpophalangeal joints, the knee joint, the shoulder joint, the wrist joint, the elbow joint, the ankle joint, the hip joint, a vertebral joint and the midfoot joints. The midfoot is the anterior part of the foot that sits between the hindfoot and forefoot. It is composed of the cuboid, navicular and three cuneiform bones. The mid-tarsal and the tarsometatarsal joints join the midfoot to the hindfoot and the forefoot respectively. The midfoot is a complex and composite area where observed oedema inflammation is generally diffuse.

In one embodiment, the present invention is dedicated to the treatment of pain in joints, in particular crystal-associated and non-crystal associated acute inflammatory arthritis, more particularly selected from non-crystal associated acute arthritis, such as Rheumatoid arthritis, Ankylosing spondylitis, Juvenile arthritis, Psoriatic arthritis, Lupus or in treating and crystal-associated arthropathies attacks or flares, such as gout, chondrocalcinosis, calcifying tendinitis and Milwaukee shoulder syndrome, and acute forms of tendinitis and capsulitis.

In one embodiment, the composition suitable for an intra-articular injection, is dedicated to the treatment of gout attack.

In one embodiment, the composition suitable for an intra-articular injection is dedicated to the treatment of chondrocalcinosis.

In one embodiment, the composition suitable for an intra-articular injection is dedicated to the treatment of acute and chronic calcifying tendinitis.

In one embodiment, the composition suitable for an intra-articular injection is dedicated to the treatment of Milwaukee shoulder syndrome.

In one embodiment, composition suitable for an intra-articular injection is dedicated to the treatment of tendinitis.

In one embodiment, composition suitable for an intra-articular injection is dedicated to the treatment of capsulitis.

The administration may be performed by a single injection or as sequential injections provided that the time required to release 80% by weight of the colchicine in the microparticles is greater than 1 day and may reach 15 days, in particular between 1.5 days and 15 days, the colchicine being present in a concentration ranging from 2.5 to 2500 µg per ml of the sterile and injectable dosage form, in particular a suspension, the anesthetic agent, in particular ropivacaine, being present in a concentration ranging from 0.05 to 120 mg per ml of the sterile and injectable dosage form, in particular a suspension, 80% of the anesthetic agent being released in maximum 3 days and the pharmaceutical composition having a volume ranging from 0.1 ml to 5 ml. In other words, the single or sequential injections, preferably the single injection, provides locally a colchicine weight ranging between 0.25 and 12500 µg of colchicine.

In one embodiment, the injection is performed a plurality of times (two times, three times, four times or more). In this embodiment, each subsequent time the injection is performed, it is performed after a suitable period has lapsed since the preceding time the injection was performed. This suitable time can be, for example, two weeks, one month, two months, three months, four months, five months, six months, one year, or longer.

In one embodiment, the colchicine is present in a concentration ranging from 2.5 to 2500 µg per ml of the sterile and injectable dosage form, in particular a suspension, in particular from 2.5 to 1500 µg per ml of the sterile and injectable dosage form, in particular a suspension, in particular from 2.5 to 1000 µg per ml, and more particularly from 2.5 to 500 µg per ml, and even more particularly from 2.5 to 250 µg per ml.

In one embodiment, the anesthetic agent is present in a concentration ranging from 0.05 to 120 mg per ml of the sterile and injectable dosage form, in particular a suspension, in particular from 0.05 to 60 mg per ml, and more particularly from 0.05 to 20 mg per ml.

In a particular embodiment, the same pharmaceutical composition, in particular under the form of a suspension, may be used for intra-articular injection to any painful joint. The amount of pharmaceutical composition to be injected may however be adapted to the targeted joint. This is one of the advantages of the invention, which may with a single concentration offer the adapted amount of colchicine and anesthetic agent for each site of application by adapting the corresponding needed volume.

In one embodiment, injections may be performed simultaneously in two or more different painful joints.

For example, the volume of pharmaceutical composition, in particular under the form of a suspension, according to the present invention that may be injected to the shoulder may vary between 2 and 5 ml.

The volume of pharmaceutical composition, in particular under the form of a suspension, according to the present invention that may be injected to the metatarsophalangeal joints, for example the toes, may vary between 0.1 and 0.5 ml, typically is 0.25 ml.

The volume of pharmaceutical composition, in particular under the form of a suspension, according to the present invention that may be injected to the metacarpophalangeal joints, for example the fingers, may vary between 0.1 and 0.5 ml, typically is 0.25 ml.

The volume of pharmaceutical composition, in particular under the form of a suspension, according to the present invention that may be injected to a vertebral joint may vary between 1 and 2 ml.

The volume of pharmaceutical composition, in particular under the form of a suspension, according to the present invention that may be injected to the midfoot joints may vary between 0.1 and 1 ml.

The volume of pharmaceutical composition, in particular under the form of a suspension, according to the present invention that may be injected to the knee, may vary between 2 and 5 ml.

The volume of pharmaceutical composition, in particular under the form of a suspension, according to the present invention that may be injected to the wrist may vary between 0.5 and 1 ml.

The volume of pharmaceutical composition, in particular under the form of a suspension according to the present invention that may be injected to the elbow may vary between 2 and 5 ml.

The volume of pharmaceutical composition, in particular under the form of a suspension, according to the present invention that may be injected to the ankle may vary between 1 and 3 ml.

The volume of pharmaceutical composition, in particular under the form of a suspension, according to the present invention that may be injected to the hip may vary between 2 and 5 ml.

In one embodiment, the pharmaceutical composition, in particular under the form of a suspension, according to the present invention may be characterized by its volume, which may be adapted to the joint, and namely may range from 2 to 5 ml for the shoulder, from 1 to 2 ml for a vertebral joint, from 0.1 to 1 ml for the midfoot joints, from 0.1 to 0.5 ml for the metatarsophalangeal joints, from 0.1 to 0.5 ml for the metacarpophalangeal joints, from 2 to 5 ml for the knee joint, from 0.5 to 1 ml for the wrist joint, from 2 to 5 ml for the elbow joint, from 1 to 3 ml for the ankle joint and from 2 to 5 ml for the hip.

It should also be understood that a specific dosage and treatment regimen for any particular patient will depend upon a variety of factors, including the age, body weight, general health, sex, diet, time of administration, drug combination, and the judgment of the treating physician and the severity of the particular disease being treated.

In one embodiment, the controlled release is suitable for reaching an articular colchicine maximum concentration (Cmax) ranging from 5 nM (2ng/mL) to 5 µM (2 µg/mL).

According to a preferred embodiment, the injection of the pharmaceutical composition of the present invention allows reaching a systemic concentration of colchicine 12 hours after the intra-articular injection which does not exceed 5 ng/mL, in particular not exceeding 2 ng/ml and more particularly not exceeding 1 ng/ml 12 hours after the intra-articular injection.

In addition, the injection of the controlled release pharmaceutical composition according to the invention can be able to achieve a synovial concentration of colchicine 12 hours after the intra-articular injection greater than 1 ng/ml, in particular comprised between 1 and 500 ng/ml, more particularly comprised between 1 and 250 ng/ml, more particularly comprised between 1 and 200 ng/ml, for example between 1 and 100 ng/ml, and even more particularly between 1 and 50 ng/ml.

The requested dose to be injected into the painful joint can depend on the type of painful joint, in particular depending on its size. Table 1a as follows gathers typical dosage of colchicine that may be implemented in the framework of the present invention.

The volume corresponds to the total volume of the composition suitable for the injection, i.e. in particular under the form of a suspension.

**Table 1a:**

| | **Joint** | **Volume (ml)** | **Drug concentration (µg/ml)** | **Dose range (µg)** |
|---|---|---|---|---|
| **Small joints** | metatarsophalangeal | 0.1-0.5 | 2.5 - 2 500 [2.5 - 250]^{∗} | 0.25 - 1 250 [0.25 - 125]^{∗} |
| | metacarpophalangeal | 0.1-0.5 | 2.5 - 2 500 _{[}2.5 - 250]^{∗} | 0.25 - 1 250 [0.25 - 125]^{∗} |
| | wrist | 0.5-1 | 2.5 - 2 500 [2.5 - 250]^{∗} | 1.25 - 2 500 [1.25 - 250]^{∗} |
| | midfoot | 0.1-1 | 2.5 - 2 500 [2.5 - 250]^{∗} | 0.25 - 2 500 [0.25 - 250]^{∗} |
| **Medium/large joints** | vertebral | 1-2 | 2.5 - 2 500 [2.5 - 250]^{∗} | 2.5 - 5 000 [2.5 - 500]^{∗} |
| | ankle | 1-3 | 2.5 - 2 500 [2.5 - 250]^{∗} | 2.5 - 7 500 [2.5 - 750]^{∗} |
| | knee | 2-5 | 2.5 - 2 500 [2.5 - 250]^{∗} | 5 - 12 500 [5 - 1 250]^{∗} |
| | hip | 2-5 | 2.5 - 2 500 [2.5 - 250]^{∗} | 5 - 12 500 [5 - 1 250]^{∗} |
| | shoulder | 2-5 | 2.5 - 2 500 [2.5 - 2 50]^{∗} | 5 - 12 500 [5 - 1 250]^{∗} |
| | elbow | 2-5 | 2.5 - 2500 [2.5 - 250]^{∗} | 5 - 12 500 [5 - 1 250]^{∗} |

| | | | | |
|---|---|---|---|---|
| ^{∗}[in particular] | | | | |

As far as the anesthetic agent is concerned, it may be suitable to reach a local concentration of said anesthetic agent, in particular of ropivacaine, between 0.05 and 50 mg/ml, in particular between 0.05 and 25 mg/ml, and even more preferentially between 0.05 and 10 mg/ml, for example between 0.05 and 3 mg/ml, as soon as the injection is performed, said local concentration being able to last 4 hours, 8 hours, 1 day, 1.5 days, 2 days or 3 days.

The requested dose to be injected into the painful joint can depend on the type of painful joint, in particular depending on its size. Table 1b as follows gathers typical dosage of anesthetic agent, in particular ropivacaine, that may be implemented in the framework of the present invention.

**Table 1b:**

| | **Joint** | **Volume (ml)** | **Drug concentration (mg/ml)** | **Dose range (mg)** |
|---|---|---|---|---|
| **Small joints** | metatarsophalangeal | 0.1-0.5 | 0.05-120 | 0.005-60 |
| | metacarpophalangeal | 0.1-0.5 | 0.05-120 | 0.005-60 |
| | wrist | 0.5-1 | 0.05-120 | 0.025-120 |
| | midfoot | 0.1-1 | 0.05-120 | 0.005-120 |
| **Medium/large joints** | vertebral | 1-2 | 0.05-120 | 0.05-240 |
| | ankle | 1-3 | 0.05-120 | 0.05-360 |
| | knee | 2-5 | 0.05-120 | 0.1-600 |
| | hip | 2-5 | 0.05-120 | 0.1-600 |
| | shoulder | 2-5 | 0.05-120 | 0.1-600 |
| | elbow | 2-5 | 0.05-120 | 0.1-600 |

In one embodiment, the pharmaceutical composition, in particular under the form of a suspension, in the present invention, which is in particular administered in one intra-articular injection, and which is dedicated to the treatment of the acute phase of crystal-associated inflammatory arthritis or non-crystal associated inflammatory arthritis or acute forms of tendinitis and capsulitis, comprises between 0.25 to 1250 µg of colchicine per injection and 0.005 to 60 mg of anesthetic agent, in particular of ropivacaine, for a small joint selected from metatarsophalangeal joint or metacarpophalangeal joint.

In another embodiment, the pharmaceutical composition, in particular under the form of a suspension, in the present invention, which is in particular administered in one intra-articular injection, and which is dedicated to the treatment of the acute phase of crystal-associated inflammatory arthritis or non-crystal associated inflammatory arthritis or acute forms of tendinitis and capsulitis, comprises between 1.25 and 2500 µg of colchicine per injection and 0.025 to 120 mg of anesthetic agent, in particular of ropivacaine, for a wrist joint.

In another embodiment, the pharmaceutical composition, in particular under the form of a suspension, in the present invention, which is in particular administered in one intra-articular injection, and which is dedicated to the treatment of the acute phase of the crystal-associated inflammatory arthritis or non-crystal associated inflammatory arthritis or acute forms of tendinitis and capsulitis, comprises between 0.25 and 2500 µg of colchicine per injection and 0.005 to 120 mg of anesthetic agent, in particular of ropivacaine, for midfoot joints.

In another embodiment, the pharmaceutical composition, in particular under the form of a suspension, in the present invention, which is in particular administered in one intra-articular injection, and which is dedicated to the treatment of the acute phase of the crystal-associated inflammatory arthritis or non-crystal associated inflammatory arthritis or acute forms of tendinitis and capsulitis, comprises between 2.5 and 7500 µg of colchicine per injection and 0.05 to 360 mg of anesthetic agent, in particular of ropivacaine, for an ankle joint or 2.5 and 5000 µg of colchicine and 0.05 to 240 mg of anesthetic agent, in particular of ropivacaine per injection in vertebral joint.

In another embodiment, the pharmaceutical composition, in particular under the form of a suspension, in the present invention, which is in particular administered in one intra-articular injection, and which is dedicated to the treatment of the acute phase of the crystal-associated inflammatory arthritis or non-crystal associated inflammatory arthritis or acute forms of tendinitis and capsulitis, comprises between 5 and 12500 µg of colchicine per injection and 0.1 to 600 mg of anesthetic agent, in particular of ropivacaine, for a joint selected from a knee joint, a hip joint, a shoulder joint and an elbow joint.

According to one embodiment, the pharmaceutical composition, in particular under the form of a suspension, in the present invention, which is in particular administered in one intra-articular injection, and which is dedicated to the treatment of the acute phase of the crystal-associated inflammatory arthritis or non-crystal associated inflammatory arthritis or acute forms of tendinitis and capsulitis, comprises between 0.25 to 2500 µg/ml, in particular 2.5 to 1500 µg/ml of colchicine and comprises between 0.05 to 120 mg/ml of anesthetic, in particular from 0.05 to 60 mg/ml, in particular of ropivacaine. In one embodiment, the pharmaceutical composition, in particular under the form of a suspension, in the present invention may for example comprise between 2.5 and 1000 µg/ml of colchicine, in particular between 2.5 and 500 µg/ml of colchicine, more particularly between 2.5 and 250 µg/ml of colchicine and comprises between 0.05 to 120 mg/ml of anesthetic, in particular between 0.05 and 60 mg/ml, in particular of ropivacaine. In one embodiment, the pharmaceutical composition, in particular under the form of a suspension, in the present invention may for example comprise between 2.5 and 500 µg/ml of colchicine, in particular between 2.5 and 250 µg/ml of colchicine and comprises between 0.05 to 60 mg of anesthetic, in particular between 0.05 and 20 mg/ml, in particular of ropivacaine.

In one embodiment, the pharmaceutical composition may be suited for maintaining a therapeutically acceptable concentration of colchicine in the painful joint during a period superior to one day to 9 days, a period superior to one day to 8 days, a period superior to one day to 7 days, a period superior to one day to 6 days, and for example during 5 days, 6 days, 7 days, 8 days, 9 days or 10 days, depending on the nature of the pathology to be treated, more particularly during 5 days.

In one embodiment, the pharmaceutical composition may be suited for maintaining a therapeutically acceptable concentration of anesthetic agent, in particular ropivacaine, in the painful joint during 3 days, 2 days or in particular from immediately after the administration to 1 day after the administration.

In one embodiment, the pharmaceutical composition for use according to the invention, is characterized by the fact that the time required to release 80% by weight of the colchicine in the microparticles is superior to 1 day, for example 1.5 day, and may reach 10 days, is superior to 1 day, for example 1.5 day, and may reach 7 days, is superior to 1 day, for example 1.5 day, and may reach 5 days, and for example is superior to 1 day, for example 1.5 day, 2 days, 3 days, 4 days, 5 days, 6 days, 7 days, 8 days, 9 days or 10 days, more particularly is superior to 1 day, for example 1.5 day, and may reach 7 days, or is comprised between 2 days and 15 days, between 2 days and 10 days and between 2 days and 7 days and wherein the time required to release 80% by weight of the anesthetic agent is maximum 3 days, for example maximum 2 days and in particular maximum 1 day.

In one embodiment, the pharmaceutical composition, in particular under the form of a suspension, in the present invention may release colchicine during 5 to 10 days and it comprises between 2.5 to 2500 µg/ml of colchicine and 0.05 to 120 mg/ml of anesthetic agent, in particular of ropivacaine, between 2.5 to 1500 µg/ml of colchicine and 0.05 to 60 mg/ml of anesthetic agent, in particular of ropivacaine or between 2.5 to 250 µg/ml of colchicine and 0.05 to 20 mg/ml of anesthetic agent, in particular of ropivacaine.

In one embodiment, the pharmaceutical composition, in particular under the form of a suspension, in the present invention may release colchicine during 6 to 9 days and it comprises between 2.5 to 2500 µg/ml or colchicine and 0.05 to 120 mg/ml of anesthetic agent, in particular of ropivacaine, in particular between 2.5 to 500 µg/ml of colchicine and 0.05 to 120 mg/ml of anesthetic agent, in particular of ropivacaine.

According to one particular embodiment, the treatment may be renewed when another acute flares arises under the same conditions and posology.

According to one embodiment, and more particularly for the treatment of acute arthritis associated with the presence of microcrystals or non-crystal associated inflammatory arthritis, and more particularly gout attacks, chondrocalcinoses and acute calcifying tendinitis, the pharmaceutical composition may be suited for providing an initial articular colchicine concentration.

The pharmaceutical composition may or may not exhibit a burst release. In the framework of the present invention a "burst release" means that an initial bolus of drug is released before the release rate reaches a stable profile, immediately upon placement in the release medium, i.e. in the present invention immediately after the injection into the articulation.

In any case, the length of colchicine burst release, may be between 0 and 1 day, for example between the beginning of day 1 through the end of day 1, and in particular is between 0 and 6 hours, between 0 and 3 hours, and even more particularly is between 0 and 2 hours.

Thus, when a colchicine burst release occurs, upon administration, the pharmaceutical composition according to the present invention may provide an initial release of colchicine at the site of administration, for example, in the intraarticular space and/or peri-articular space. Once the initial release of colchicine has subsided, the controlled release of the colchicine microparticle formulation continues to provide therapeutic (e.g., intra-articular and/or peri-articular) concentrations of colchicine to combat inflammation and/or pain for an additional period of therapy following administration.

In a particular embodiment, the pharmaceutical composition according to the present invention does not exhibit an important colchicine burst release. According to said embodiment, less than 50% by weight of colchicine is released at 12 hours after the administration. This is in particular one of the distinguishing characteristics in Aboumanei *et al*, as discussed in the preamble.

According to another aspect, the pharmaceutical composition for use according to the present invention may be further characterized in that less than 50% by weight of colchicine is released at 12 hours after the administration.

According to another aspect the pharmaceutical composition for use according to the present invention may be further characterized in that said pharmaceutical composition presents an *in vitro* dissolution profile, wherein less than 25% of colchicine is released within 60 minutes, less than 50% of colchicine is released in 12 hours and wherein at least 80% of the anesthetic agent is released within 3 days, within 2 days or within 1 day.

According to another aspect, the invention relates to a method for the treatment of crystal-associated and non-crystal associated acute inflammatory arthritis, in particular a non-crystal associated acute arthritis, such as Rheumatoid arthritis, Ankylosing spondylitis, Juvenile arthritis, Psoriatic arthritis, Lupus or a crystal-associated arthropathy attacks or flares or acute forms of tendinitis and capsulitis comprising at least the administering by injection to a painful joint to a patient in need thereof of a pharmaceutical composition comprising an anesthetic agent and controlled release dosage form comprising an effective amount of colchicine, suitable for intra-articular injection into a joint, wherein the time required to release 80% by weight of the colchicine is greater than 1 day and may reach 15 days, in particular is comprised between 1.5 days and 15 days, wherein the colchicine is present in a concentration ranging from 2.5 to 2500 µg per ml of the sterile and injectable dosage form, in particular a suspension, and wherein the anesthetic agent, in particular ropivacaine, is present in a concentration ranging from 0.05 to 120 mg per ml of the sterile and injectable dosage form, in particular a suspension, wherein 80% of the anesthetic agent is released in maximum 3 day and wherein the pharmaceutical composition has a volume ranging from 0.1 ml to 5 ml.

Still according to another aspect, the invention relates to a method for the treatment of crystal-associated and non-crystal associated acute inflammatory arthritis, in particular a non-crystal associated acute arthritis, such as Rheumatoid arthritis, Ankylosing spondylitis, Juvenile arthritis, Psoriatic arthritis, Lupus or a crystal-associated arthropathy attacks or flares or acute forms of tendinitis and capsulitis comprising at least the administration by injection into the joints of a patient in need thereof of at least an efficient amount of an anesthetic agent and at least an efficient amount of colchicine for a controlled release.

Herein is further provided a method for the treatment of crystal-associated and non-crystal associated acute inflammatory arthritis, in particular of crystal-associated arthropathy attacks or flares or acute forms of tendinitis and capsulitis comprising at least the steps of:
- preparing and/or providing a pharmaceutical composition under the form of a sterile and injectable dosage form, in particular a suspension, by mixing a controlled release dosage form comprising colchicine as detailed herein after or a formulation under the form of a powder as described herein after, with an aqueous injection vehicle, wherein the pharmaceutical composition optionally comprises an excipient selected from a group consisting of a tonicity-enhancing agent, a wetting-agent, a viscosity-enhancing agent, a density-enhancing agent or a mixture thereof, and wherein said excipient may be present in the aqueous injection vehicle or in the controlled release dosage form comprising colchicine or powder, and
- injecting said pharmaceutical composition into a painful joint of a patient in need thereof, wherein the volume to be injected is adapted to the injection site.

The present invention may be particularly suitable for treating patients having renal and/or hepatic impairment of patients being simultaneously treated with drugs. Indeed, for patients having renal and/or hepatic impairment, adjustments of the recommended oral dosage of colchicine, in particular in the treatment of gout flares; are required of adaptation of the administration scheme. Moreover, some drugs have been identified in the past with oral colchicine treatments as generating undesirable side effects and even fatal drug interactions, drastically modifying the safety profile and necessitating oral colchicine dosage adjustments which may for example require a reduction by two to three times of the oral colchicine dosage.

Thus, in one embodiment, the pharmaceutical composition conform to the present invention may be administered to patients having renal and/or hepatic impairment. In particular, colchicine plasmatic level is deemed to rise to toxicity threshold when intrinsic factor renal or hepatic impairment achieve Severe grade.

Patients having renal impairment may be identified and classified by techniques known to the physicians such as *via* the Creatinine Clearance method or *via* the Glomerular Filtration Rate (GFR) method. Said methods are in particular detailed in Worboys PD, Wong SL, Barriere SL. Pharmacokinetics of intravenous telavancin in healthy subjects with varying degrees of renal impairment. Eur J Clin Pharmacol. 2015 Jun;71(6):707-714. The use and method according to the present invention may for example be implemented to treat patients having all grades of renal impairment such as grades 1 to 5 as set by the GFR method. The use and method according to the present invention may be more particularly suited for patients having high grades of renal impairment, such as grades 3 to 5 as set by the GFR method.

Patients having hepatic impairment may be identified and classified by techniques known to the physicians such as *via* the Score of Child-pugh method or *via* the MELD score. Said methods are in particular detailed in Tsoris A, Marlar CA. Use Of The Child Pugh Score In Liver Disease. 2021 Mar 22. In: StatPearls [Internet]. Treasure Island (FL): StatPearls Publishing; 2022 Jan-. PMID: 31194448 or on internet under the following links: https://liverfellow.org/post/meld-score-part1 and https://liverfellow.org/post/meld- score-part2. The use and method according to the present invention may for example be implemented to treat patients having all scores, rates and/or classification, for example mild, moderate of severe hepatic impairment as set by the Child-Pugh method. The use and method according to the present invention may be more particularly suited for patients having high scores, rates and/or classification of severe hepatic impairment as set by the Child-Pugh method such as moderate and severe hepatic impairment.

Thus, due to the colchicine systemic plasmatic level being maintained under the threshold leading to toxicity in the framework of the present invention, no colchicine dosage adjustment is required even for treating patients with intrinsic factor impacts, such as renal and/or hepatic impairment that currently require colchicine dosage adjustments when orally administered.

Thus, in one embodiment, the pharmaceutical composition for use according to the present invention may further be characterized by the fact that the patient to which said pharmaceutical composition is administered have renal and/or hepatic impairment.

In one other embodiment, further provided a method for the treatment of crystal-associated and non-crystal associated acute inflammatory arthritis, in particular a non-crystal associated acute arthritis, such as Rheumatoid arthritis, Ankylosing spondylitis, Juvenile arthritis, Psoriatic arthritis, Lupus or a crystal-associated arthropathy attacks or flares or acute forms of tendinitis and capsulitis as defined herein above to a patient having renal and/or hepatic impairment, in particular wherein the dosage of colchicine is not reduced in response to such impairments.

In one embodiment, the pharmaceutical composition conform to the present invention may be co-administered with a wide range of drugs as detailed herein after, in particular showing improved safety profiles in comparison to the classical treatment with oral colchicine when co-administered with such drugs.

As an example of drug interaction that the present invention allows to avoid are the interactions with drugs that have been clearly identified in the framework of treatments with oral colchicine and that are well known to the physicians. In a situation of potential drug interaction, the classical way to proceed consists in adjusting, namely lowering the dosage of oral colchicine, depending on the severity of the interaction, according to well-known recommendations.

Such drug drug interactions (DDI) may be explained by the fact that some drugs inhibit P-gp and/or CYP3A4, generating peaks of excessive plasmatic concentration of colchicine, and in particular peaks above 6 ng/mL of colchicine, that must be avoided.

One advantage of the present invention relies in the fact that excessive plasmatic concentration is avoided and allows co-administration of drugs without adjusting the colchicine dosage it must be done when orally administered.

Thus, in one embodiment, the pharmaceutical composition for use according to the present invention may further be characterized by the fact that the patient to which said pharmaceutical composition is administered may simultaneously, separately or sequentially be treated by the following active ingredients: atazanavir, clarithromycin, darunavir, ritonavir, indinavir, itraconazole, ketoconazole, lopinavir, ritonavir, nefazodone, nelfinavir, ritonavir, saquinavir, telithromycin, tipranavir, amprenavir, aprepitant, diltiazem, erythromycin, fluconazole, fosamprenavir, verapamil, cyclosporine and ranolazine.

Herein is thus provided the pharmaceutical composition for use as defined above wherein it is for use in the treatment of crystal-associated and non-crystal associated acute inflammatory arthritis, in particular a crystal-associated arthropathy or flares or acute forms of tendinitis and capsulitis in a patient being simultaneously, separately or sequentially and non-exclusively treated by at least one of the active ingredient selected from: atazanavir, clarithromycin, darunavir, ritonavir, indinavir, itraconazole, ketoconazole, lopinavir, nefazodone, nelfinavir, saquinavir, telithromycin, tipranavir, amprenavir, aprepitant, diltiazem, erythromycin, fluconazole, fosamprenavir, verapamil, cyclosporine, ranolazine, macrolides and statins.

Herein is thus further provided a method for the treatment of crystal-associated and non-crystal associated acute inflammatory arthritis, in particular a non-crystal associated acute arthritis, such as Rheumatoid arthritis, Ankylosing spondylitis, Juvenile arthritis, Psoriatic arthritis, Lupus or a crystal-associated arthropathy attacks or flares or acute forms of tendinitis and capsulitis as defined herein above to a patient being simultaneously, separately or sequentially treated by at least one active ingredient selected from: atazanavir, clarithromycin, darunavir, ritonavir, indinavir, itraconazole, ketoconazole, lopinavir, ritonavir, nefazodone, nelfinavir, ritonavir, saquinavir, telithromycin, tipranavir, amprenavir, aprepitant, diltiazem, erythromycin, fluconazole, fosamprenavir, verapamil, cyclosporine and ranolazine, in particular wherein the dosage of colchicine is not reduced in response to the concomitant administration of such drug.

### CONTROLLED RELEASE DOSAGE FORM COMPRISING COLCHICINE

According to one embodiment, the controlled release dosage form comprising colchicine is under the form of an in-situ forming depot, a hydrogel or microparticles, in particular microparticles comprising a polymeric matrix or multivesicular liposomes.

In one embodiment, the controlled release dosage form comprising colchicine is under the form of an in-situ forming depot. An in-situ forming depot, such as pH-induced, thermally-induced or solvent exchange-induced gelling systems comprising colchicine may be prepared according to techniques known to the man skilled in the art, as in particular described in L. Rahnfeld et al. Injectable Lipid-Based Depot Formulations: Where Do We Stand?. Pharmaceutics. 2020, 12(6):567, in S. Kempe et al. In-situ forming implants-an attractive formulation principle for parenteral depot formulations Journal of Controlled Release 2012, 161:668.

In another embodiment, the controlled release dosage form comprising colchicine is under the form of a hydrogel. A hydrogel may be prepared according to techniques known by the man skilled in the art. In particular a hydrogel may be formed by dispersing the colchicine, optionally previously solubilized in water, under stirring with a hydrogel. Among suitable hydrogels, the following may be cited: sodium hyaluronate. When the pharmaceutical composition is under the form of a powder, water shall be removed by techniques known to the man skilled in the art, for example by dehydration or lyophilization. When needed they may be hydrated back reversibly.

In one embodiment, the controlled release dosage form comprising colchicine is under the form of microparticles. Said embodiments are described in detail herein after.

### CONTROLLED RELEASE MICROPARTICLES COMPRISING COLCHICINE

The controlled release microparticles comprising colchicine may take the form of various microparticles, such as (i) microparticles comprising a polymeric matrix or (ii) multivesicular liposomes.

According to one embodiment, the microparticles have a mean particle size equal or greater than 10 µm.

According to another embodiment, the microparticles have a mean particle size equal or greater than 10 µm, in particular a mean particle size lower than 100 µm, more particularly lower than 80 µm, and even more more particularly lower than 50 µm, for example between 10 and 50 µm or between 10 and 40 µm.

It is understood that these ranges refer to the mean size of all microparticles in a given population. The size of any given individual microparticle could be within a standard deviation above or below the mean size.

Within the context of the present invention, a "microparticle" means a particle of any shape and made of any material, in particular suitable for the injection into the human body within a pharmaceutical composition, and in particular into the articulations or joints, having a mean particle size greater than 10 µm and less than 100 µm.

The microparticles suitable for the pharmaceutical composition used according to the present invention may be selected form various types of microparticles such as microspheres, microparticle matrices, microsphere matrices, microcapsules, rods, wafers, pills fibers and pellets.

### (i) MICROPARTICLES COMPRISING A POLYMERIC MATRIX

The polymeric matrix may be chosen from various polymers suitable for obtaining the controlled release microparticles. Such polymeric matrix is nontoxic to the human body.

According to one embodiment, the polymer forming the polymeric matrix is biodegradable.

Within the context of the present invention, a "biodegradable" material, refers to a material which degrades enzymatically or hydrolytically and for which there is proof that the degradation products are integrated into biomass and/or eliminated from the organism by metabolism or renal filtration.

The nontoxic and biodegradable polymer may be natural or synthetic.

According to one embodiment, the polymeric matrix of the microparticles used in the pharmaceutical composition used according to the present invention comprises at least a poly(lactic-co-glycolic acid) copolymer or PLGA.

According to said embodiment, the polymeric matrix may comprise PLGA in an amount of greater than 70% by weight, with respect to the total weight of the polymeric matrix, in particular greater than 80% by weight, and even more particularly greater than 90% by weight.

Suitable polymers are not limited to those commercially available and known as RESOMER (Evonik Industries AG, Germany), LACTEL (Durect, USA), PURASORB (Corbion NV, The Netherlands), Viatel (Ashland, USA), EXPANSORB (Seqens, FRANCE).

Examples of suitable polymers are listed in Table 2.

**Table 2:**

| **Examples of suitable polymers** | | | |
|---|---|---|---|
| **Product name** | **Polymer** | **End group** | **Producer** |
| Resomer RG 502H | Polv(D,L-lactide-co-glvcolide) 50:50 | acid | Evonik |
| Resomer RG 502 | Polv(D,L-lactide-co-glvcolide) 50:50 | ester | Evonik |
| Resomer RG 503H | Poly(D,L-lactide-co-glycolide) 50:50 | acid | Evonik |
| Resomer RG 503 | Polv(D,L-lactide-co-glvcolide) 50:50 | ester | Evonik |
| Lactel B6013-1 | Polv(D,L-lactide-co-glvcolide) 50:50 | acid | Durect |
| Lactel B6017-1 | Polv(D,L-lactide-co-glvcolide) 50:50 | ester | Durect |
| Lactel B6010-1 | Polv(D,L-lactide-co-glvcolide) 50:50 | ester | Durect |
| Purasorb PDLG 5002A | Polv(D,L-lactide-co-glvcolide) 50:50 | acid | Corbion |
| Purasorb PDLG 5002 | Poly(D,L-lactide-co-glycolide) 50:50 | ester | Corbion |
| Purasorb PDLG 5004A | Polv(D,L-lactide-co-glvcolide) 50:50 | acid | Corbion |
| Purasorb PDLG 5004 | Poly(D,L-lactide-co-glycolide) 50:50 | ester | Corbion |
| Viatel | Polv(D,L-lactide-co-glvcolide) 50:50 | acid/ester | Ashland |
| Expansorb DLG 2A | Polv(D,L-lactide-co-glvcolide) 50:50 | acid | Seqens |
| Expansorb DLG 3A | Polv(D,L-lactide-co-glvcolide) 50:50 | acid | Seqens |
| Expansorb DLG 5A | Poly(D,L-lactide-co-glycolide) 50:50 | acid | Seqens |
| Expansorb DLG 6A | Polv(D,L-lactide-co-glvcolide) 50:50 | acid | Seqens |
| Expansorb DLG 2E | Poly(D,L-lactide-co-glycolide) 50:50 | ester | Seqens |
| Expansorb DLG 6E | Poly(D,L-lactide-co-glycolide) 50:50 | ester | Seqens |

According to another embodiment, the polymeric matrix of the microparticles used in the pharmaceutical composition used according to the present invention comprises at least a poly(caprolactone) (PCL).

According to said embodiment, the polymeric matrix may comprise PCL in an amount of greater than 70% by weight, with respect to the total weight of the polymeric matrix, in particular greater than 80% by weight, and even more particularly greater than 90% by weight.

According to another embodiment, the polymeric matrix of the microparticles used in the pharmaceutical composition according to the present invention comprises a mixture of at least a PCL and at least a PLGA.

In a particular embodiment, the pharmaceutical composition according to the present invention, is characterized by the microparticles, which are microparticles comprising colchicine and a polymeric matrix, wherein the polymeric matrix comprises at least a poly(lactic-co-glycolic acid) copolymer, at least poly(caprolactone) or at least a mixture of poly(lactic-co-glycolic acid) copolymer and poly(caprolactone).

In one embodiment, the polymeric matrix comprises poly(lactic-co-glycolic acid), copolymer poly(caprolactone),or a mixture of poly(lactic-co-glycolic acid) copolymer and poly(caprolactone) in an amount of greater than 70% by weight, with respect to the total weight of the polymeric matrix, in particular greater than 80% by weight, and even more particularly greater than 90% by weight.

According to said embodiment, the polymeric matrix may comprise a mixture of PCL and PLGA in an amount of greater than 70% by weight, with respect to the total weight of the polymeric matrix, in particular greater than 80% by weight, and even more particularly greater than 90% by weight.

The polymeric matrix may comprise one or more additional polymer(s), copolymer(s) or mixture thereof. Said additional polymer(s), copolymer(s) or mixture thereof may be present in the polymer matrix in an amount ranging from 0 to 30% by weight, in particular from 0 to 20% by weight, and more particularly from 0 to 10% by weight, with respect to the total weight of the polymeric matrix.

Non-limiting examples of suitable additional polymers or copolymers include poly(lactide) or PLA distinct from poly(lactic-co-glycolic acid) copolymer, poly(glycolide) or PGA distinct from poly(lactic-co-glycolic acid) copolymer, poly(lactide-co-caprolactone), poly(ethylene glycol), poly(ethylene oxide) or PEO, PLGA-b-PEO-b-PLGA, PLGA-b-PEO, polyhydroxyalkanoates, poly(hydroxybutyrate), poly(trimethylene carbonate), poly(dioxanone), poly(valerolactone), poly(alpha- hydroxy acids), poly(lactones), poly(amino acids), polyanhydrides, poly(orthoester), poly(acetals), polyurethanes, polythioesters, polyphosphoesters, poly(ester-co-amide), poly(vinyl alcohol) or PVA, PVA-g-PLGA, poly (ether ester) multiblock copolymers, polyvinyl pyrrolidone, poly(methacrylate), PEO-PPO-PEO (pluronics), gelatin, heparin, chondroitin sulfate; polysaccharides such as alginates, starch, chitosan and dextrans and any combinations thereof.

In one embodiment, the controlled release microparticles according to the present invention are PLGA microspheres. In other words, in said embodiment, the polymeric matrix does not comprise any additional polymer or copolymer.

When PLGA copolymers are implemented as a polymer matrix, they can have a wide range of molecular weights and monomer ratios of lactic to glycolic acid, in particular of 75:25 to 50:50, and even more particularly of 50:50. Any suitable method known in the art of making the polymer can be used and the molecular weights may typically range from 5 to 150 kDa, in particular from 5 to 80 kDa, and more particularly from 5 to 50 kDa.

According to a further particular embodiment, the polymer forming the polymeric matrix comprises PLGA, for example in an amount of 100% by weight, with respect to the total weight of the polymeric matrix, and the PLGA is defined by:
- A molecular weight between 5 and 80 kDa, and
- A lactide:glycolide molar ratio of 75:25 to 50:50.

According to a more particular embodiment, the polymer forming the polymeric matrix comprises PLGA, for example in an amount of 100% by weight, with respect to the total weight of the polymeric matrix, and the PLGA is defined by:
- A molecular weight between 5 and 50 kDa, and
- A lactide:glycolide molar ratio of 50:50.

According to one embodiment, PLGA is either carboxylic, ester or PEG endcapped, and is in particular carboxylic endcapped.

According to another embodiment, microparticle matrix can comprise, and even may consist in, a blend of two PLGA copolymers, in particular one of low molecular weight and one of high molecular weight.

The microparticle may further comprise pharmaceutically acceptable excipients for modulating the drug release profile such as medium chain triglycerides, poly(oxyethylene) sorbitan fatty acid esters (e.g. polysorbate 20, polysorbate 80), sorbitan fatty acid esters, cyclodextrins, lecithin, mannitol, sucrose, inorganic salts and mixtures thereof.

The polymeric matrix of the microparticles may comprise one or more excipient(s). Said excipient(s) may be present in the polymer matrix in an amount not exceeding 15% by weight, in particular not exceeding 10% by weight, and more particularly not exceeding 5% by weight, with respect to the total weight of the polymeric matrix.

In one embodiment, the percentage weight ratio between the colchicine and the total weight of the microparticles or drug loading content in the pharmaceutical composition ranges from 0.1 to 35% by weight, in particular from 0.5 to 30% by weight, more particularly from 1 to 25% by weight, and even more particularly from 1 to 10% by weight.

### Manufacturing process for the obtention of the microparticles comprising a polymer matrix

Any process suitable to produce polymeric microparticles with a mean particle size ranging from 10 µm to 100 µm is considered appropriate in the framework of the present invention.

Among such manufacturing process emulsification-based processes, such as high-pressure homogenization, for example using rotor-stator homogenizer in a batch or continuous mode, or membrane emulsification, followed by organic solvent removal by extraction/evaporation may be cited.

As far as the general principal of such methods is concerned, an emulsion may be prepared and processed over a membrane with pores with a determined size. The obtained microspheres can then be collected after extraction and evaporation of the organic solvent, washed and freeze-dried.

According to one particular embodiment, the microparticles may be manufactured from an O/W direct emulsion or from a W/O/W double emulsion technique.

Schoubben, A., Ricci, M. & Giovagnoli, S. Meeting the unmet: from traditional to cutting-edge techniques for poly lactide and poly lactide-co-glycolide microparticle manufacturing. J. Pharm. Investig. 49, 381-404 (2019) reviews various methods to make PLGA microparticles that can be used in the framework of the present invention.

According to one particular embodiment, the microparticles may be manufactured from a Solid-in-Oil-in-Water (S/O/W) double emulsion technique.

Giovagnoli, S., et al.; Physicochemical characterization and release mechanism of a novel prednisone biodegradable microsphere formulation, J Pharm Sci. 97:303-317, (2008) describes an example of PLGA microparticles prepared *via* S/O/W emulsion technique. Other manufacturing approaches suitable for obtaining the microparticles according to the present invention are atomization by spinning disk, atomization by spray-drying, a fluidized bed coating, or combinations thereof.

Alternatively, the microparticles may be manufactured using drop-on-demand, drop-by-drop and jet break-up processes such as inkjet printing or microfluidics.

Alternatively, the microparticles may be manufactured using supercritical fluid technologies.

Alternatively, the microparticles may be manufactured using microfabrication methods, such as template and mold-based techniques, such as soft lithography.

All these manufacturing processes are well known by the man skilled in the art.

The hereabove cited manufacturing processes are well known to the man skilled in the art.

### (ii) MULTI VESICULAR LIPOSOMES

Multivesicular liposomes (MVL) are spherical particles with a mean diameter of 10-30 µm and composed of non-concentric multiple lipid bilayers arranged in a honeycomblike structure. These lipid layers enclose numerous water-filled aqueous compartments that can be used to encapsulate a water-soluble drug such as colchicine.

MVLs are classically composed of at least one amphiphilic lipid and one neutral lipid. The amphiphilic lipid is selected from phospholipids for instance phosphatidylcholine or phosphatidylglycerol. The neutral lipid is selected from triglycerides having mono-unsaturated fatty acid ester moieties containing 14-18 carbons in the acyl chain (e.g. triolein, tripalmitolein), saturated fatty acid ester moieties containing 6 to 8 carbons in the acyl chain (e.g. tricaproin, tricaprylin) and mixtures thereof. Cholesterol can also be used in the composition.

MVLs are obtained by using a water-in-oil-in-water double emulsification process. In a first step, a water-in-oil emulsion is prepared by mixing phospholipids, triolein, tricaprylin and cholesterol solubilized in volatile and water-immiscible organic solvent with an aqueous solution containing the solubilized drug to be encapsulated. This first emulsion is then emulsified by mixing with a second aqueous solution to produce the water-in-oil-in-water emulsion. The energy needed to form the first and second emulsion can be provided either mechanically, by sonication or by combinations thereof. MVLs are finally obtained by removal of the volatile organic solvent from the double emulsion using gas stripping or flushing. Finally, removal of unencapsulated material, concentration of the MVLs and buffer exchange is performed using either diafiltration or cross-flow filtration system.

In one embodiment, the neutral lipids used to manufacture the MVL encapsulating colchicine comprises a mixture of triolein : tricaprylin with ratios ranging from 50:50 to 0:100.

### CONTROLLED RELEASE FORMS COMPRISING THE ANESTHETIC AGENT

In one embodiment, the pharmaceutical composition is characterized in that the anesthetic agent is partially or totally under an immediate release form or is partially or totally under a controlled release form, said controlled release form being distinct from the controlled release dosage form comprising colchicine or totally of partially being comprised in it, such as under the form of microparticles comprising a polymeric matrix, in particular as defined above for colchicine, under the form of a hydrogel, under the form of multivesicular liposomes or under the form of an in-situ forming depot.

In other words, in the framework of the present invention, when the anesthetic agent presents a part of it being under the form of a controlled release form, said part may be distinct from the controlled release form comprising colchicine or form part of it, i.e. said part of anesthetic agent may be present in the controlled release dosage form comprising colchicine, for example in microparticles comprising colchicine.

The anesthetic agent may be under the form of controlled release microparticles such as (i) microparticles comprising a polymeric matrix obtained either *via* Oil-in-Water emulsion (O/W) or *via* Water-in-Oil-in-Water emulsion (W/O/W) or *via* Solid-in-Oil-in Water emulsion (S/O/W), (ii) multivesicular liposomes, (iii) dehydrated hydrogel or (iv) in-situ forming depot, in particular obtained from pH-induced, thermally-induced or solvent exchange-induced gelling systems.

In one embodiment, the (i) controlled release microparticles comprising the anesthetic agent may be prepared according the same characteristics and methods as described above for the preparation of controlled release microparticles comprising colchicine. In particular, the same size and nature of polymeric matrix may be used.

In particular, said microparticles comprising the anesthetic agent may have a polymeric matrix, wherein the polymeric matrix comprises at least a poly(lactic-co-glycolic acid) copolymer, at least poly(caprolactone) or at least a mixture of poly(lactic-co-glycolic acid) copolymer and poly(caprolactone).

In addition, said microparticles comprising the anesthetic agent may have a mean particle size equal or greater than 10 µm. Said microparticles may have a mean particle size lower than 100 µm, in particular lower than 80 µm, and more particularly lower than 50 µm, for example between 10 and 50 µm or between 10 and 40 µm.

In one embodiment, the polymeric matrix of the microparticles comprising an anesthetic agent used in the pharmaceutical composition used according to the present invention comprises at least a poly(lactic-co-glycolic acid) copolymer or PLGA.

According to said embodiment, the polymeric matrix of the microparticles comprising the anesthetic agent may comprise PLGA in an amount of greater than 70% by weight, with respect to the total weight of the polymeric matrix, in particular greater than 80% by weight, and even more particularly greater than 90% by weight.

According to another embodiment, the polymeric matrix of the microparticles comprising the anesthetic used in the pharmaceutical composition according to the present invention comprises at least a poly(caprolactone) (PCL). All the other polymers as listed in table 2 above may also be used for preparing the microparticles comprising an anesthetic agent.

According to said embodiment, the polymeric matrix may comprise PCL in an amount of greater than 70% by weight, with respect to the total weight of the polymeric matrix, in particular greater than 80% by weight, and even more particularly greater than 90% by weight.

According to another embodiment, the polymeric matrix of the microparticles comprising an anesthetic agent used in the pharmaceutical composition according to the present invention comprises a mixture of at least a PCL and at least a PLGA.

In a particular embodiment, the pharmaceutical composition according to the present invention, is characterized by the fact that the pharmaceutical composition comprises microparticles comprising the anesthetic agent, which are microparticles comprising the anesthetic agent and a polymeric matrix, wherein the polymeric matrix comprises at least a poly(lactic-co-glycolic acid) copolymer, at least poly(caprolactone) or at least a mixture of poly(lactic-co-glycolic acid) copolymer and poly(caprolactone).

In one embodiment, the polymeric matrix comprises poly(lactic-co-glycolic acid), copolymer poly(caprolactone) or a mixture of poly(lactic-co-glycolic acid) copolymer and poly(caprolactone) in an amount of greater than 70% by weight, with respect to the total weight of the polymeric matrix, in particular greater than 80% by weight, and even more particularly greater than 90% by weight.

According to said embodiment, the polymeric matrix may comprise a mixture of PCL and PLGA in an amount of greater than 70% by weight, with respect to the total weight of the polymeric matrix, in particular greater than 80% by weight, and even more particularly greater than 90% by weight.

The microparticles comprising an anesthetic agent may also comprise any suitable additional polymer and/or acceptable excipients as described above for the preparation of microparticles comprising colchicine.

According to one embodiment, the drug loading content or percentage weight ratio between the anesthetic agent and total weight of the microparticles ranges from 1% to 40%, in particular from 5 to 40%, even more particularly from 5 to 30%, for example from 10 to 30%.

In one embodiment, the controlled release microparticles comprising the anesthetic agent according to the present invention are PLGA microspheres. In other words, in said embodiment, the polymeric matrix does not comprise any additional polymer or copolymer.

When PLGA copolymers are implemented as a polymer matrix, they can have a wide range of molecular weights and monomer ratios of lactic to glycolic acid, in particular of 75:25 to 50:50, and even more particularly of 50:50. Any suitable method known in the art of making the polymer can be used and the molecular weights may typically range from 5 to 150 kDa, in particular from 5 to 80 kDa, and more particularly from 5 to 50 kDa.

According to a further particular embodiment, the polymer forming the polymeric matrix comprises PLGA, for example in an amount of 100% by weight, with respect to the total weight of the polymeric matrix, and the PLGA is defined by:
- A molecular weight between 5 and 80 kDa, and
- A lactide:glycolide molar ratio of 75:25 to 50:50.

According to a more particular embodiment, the polymer forming the polymeric matrix comprises PLGA, for example in an amount of 100% by weight, with respect to the total weight of the polymeric matrix, and the PLGA is defined by:
- A molecular weight between 5 and 50 kDa, and
- A lactide:glycolide molar ratio of 50:50.

According to one embodiment, PLGA is either carboxylic or ester endcapped, and is in particular carboxylic endcapped.

According to another embodiment, microparticle matrix comprising an anesthetic agent can comprise, and even may consist in, a blend of two PLGA copolymers, in particular one of low molecular weight and one of high molecular weight.

In one other embodiment, the (ii) multivesicular liposomes comprising the anesthetic agent may be prepared according the same characteristics and methods as described above for the preparation of multivesicular liposomes comprising colchicine.

In one other embodiment, the (iii) dehydrated hydrogel comprising the anesthetic agent may be prepared according to techniques known to the man skilled in the art. For example, the hydrogel may be achieved according to the method as described in example 5. A gel may be formed by dispersing the anesthetic agent, optionally previously solubilized in water, under stirring with a hydrogel. Among suitable hydrogels, the following may be cited: sodium hyaluronate. When the pharmaceutical composition is under the form of a powder, water shall be removed by techniques known to the man skilled in the art, for example by dehydration or lyophilization. When needed they may be hydrated back reversibly.

In one other embodiment, the (iv) in-situ forming depot such as pH-induced, thermally-induced or solvent exchange-induced gelling systems comprising the anesthetic agent may be prepared according to techniques known to the man skilled in the art, as in particular described in L. Rahnfeld et al. Injectable Lipid-Based Depot Formulations: Where Do We Stand?. Pharmaceutics. 2020, 12(6):567 , in S. Kempe et al. In-situ forming implants-an attractive formulation principle for parenteral depot formulations Journal of Controlled Release 2012, 161:668 and in K.S. Oh et al. Preclinical studies of ropivacaine extended-release from a temperature responsive hydrogel for prolonged relief of pain at the surgical wound. Int. J. of. Pharmaceutics. 2019, 558 (225-230).

### PHARMACEUTICAL COMPOSITIONS AND KITS

As mentioned above, herein are provided pharmaceutical compositions in various forms, i.e. under the form of a solution, a suspension, a solid implant, a semi-solid implant, a powder and an in-situ forming depot. Further pharmaceutical compositions are provided under the form of a powder or under the form of a kit. When under the form of a powder, the pharmaceutical composition is mainly aimed for storage whereas the solution, suspension, solid implant, semi-solid implant, powder or in-situ depot, in particular the the suspension, is the ready to use composition, ready for injection and the kit allows to store separately (i) an aqueous injection vehicle and (ii) a controlled release dosage form comprising colchicine, in particular under the form of a powder for forming a sterile and injectable dosage form, in particular a suspension, suitable for the injection.

In one embodiment, the pharmaceutical composition is further characterized in that it is in particular under the form of a sterile and injectable suspension, optionally comprising an excipient selected from a group consisting of a tonicity-enhancing agent, a wetting-agent, a viscosity-enhancing agent, a density-enhancing agent or a mixture thereof, and wherein it is obtained by mixing controlled release dosage form comprising colchicine, in particular microparticles comprising colchicine, more particularly as defined above with an aqueous injection vehicle and an anesthetic agent, in particular as defined above.

Various embodiments of said alternative forms are detailed herein after.

Solution, solid implant, semi-solid implant, powder and in-situ forming depot may be prepared according to methods known to the man skilled in the art.

According to a particular embodiment, the sterile and injectable dosage form is under the form of a suspension that may be obtained from a powder, as detailed herein after.

### Powder and suspension

In one embodiment is provided a pharmaceutical composition under the form of a powder comprising an anesthetic agent and controlled release dosage form comprising colchicine, wherein said controlled release dosage form comprising colchicine are under the form of microparticles, in particular microparticles comprising a polymeric matrix, more particularly having a mean particle size equal or greater than 10 µm, and even more particularly as defined above, said anesthetic agent being in particular as defined above.

In one embodiment is provided a pharmaceutical composition under the form of a powder comprising an anesthetic agent and controlled release microparticles comprising colchicine, wherein the microparticles are microparticles comprising a polymeric matrix or multivesicular liposomes, said microparticles comprising colchicine having a mean particle size equal or greater than 10 µm.

In another embodiment is provided a pharmaceutical composition under the form of a sterile and injectable suspension suitable for an intra-articular injection, obtained by mixing a composition under the form of a powder according to the invention, with an aqueous injection vehicle, wherein the pharmaceutical composition optionally comprises an excipient selected from a group consisting of a tonicity-enhancing agent, a wetting-agent, a viscosity-enhancing agent, a density-enhancing agent or a mixture thereof, and wherein said excipient may be present in the aqueous injection vehicle or in the powder.

According to one embodiment, the powder further comprises an immediate release dosage form containing colchicine, in particular wherein the maximal weight ratio of immediate release colchicine relative to the total amount of colchicine is 25%.

When the pharmaceutical composition according to the invention is under the form of a powder and the immediate release form of anesthetic agent as mentioned above may be mixed with the powder comprising the colchicine, prepared as described above.

Still when the pharmaceutical composition is under the form of a powder, for achieving sustained release, the anesthetic agent may be present under the form of (i) microparticles comprising a polymeric matrix obtained either *via* Oil-in-Water emulsion (O/W) or *via* Water-in-Oil-in-Water emulsion (W/O/W) or *via* Solid-in-Oil-in Water emulsion (S/O/W), (ii) multivesicular liposomes, (iii) dehydrated hydrogel or (iv) in-situ forming depot in particular obtained from pH-induced, thermally-induced or solvent exchange-induced gelling systems.

In one embodiment, the anesthetic agent is present under the form of controlled release microparticles as described in more details herein above.

The immediate release form of anesthetic agent as mentioned above may be directly solubilized into the suspension according to the invention, which is dedicated to the be administered to the patient by intra-articular injection. It means that the anesthetic agent is not comprised in the colchicine microparticles.

In one embodiment, the anesthetic agent may be present directly in the sterile and injectable dosage form, in particular a suspension, according to the present invention in particular in an amount adapted to stay below its solubility limit.

In such an embodiment, the controlled release dosage form comprising colchicine, which is under the form of an in-situ forming depot, a hydrogel or microparticles, in particular microparticles comprising a polymeric matrix or multivesicular liposomes, and the controlled release form comprising the anesthetic agent, in particular microparticles comprising the anesthetic agent, are distinct and are formulated together in a unique sterile and injectable dosage form, in particular a suspension, or pharmaceutical composition according to the present invention.

The present invention further relates to a pharmaceutical composition under the form of a sterile and injectable suspension suitable for an intra-articular injection, obtained by mixing a formulation under the form of a powder as described above, with an aqueous injection vehicle, wherein the pharmaceutical composition optionally comprises an excipient selected from a group consisting of a tonicity-enhancing agent, a wetting-agent, a viscosity-enhancing agent, a density-enhancing agent or a mixture thereof, and wherein said excipient may be present in the aqueous injection vehicle or in the powder.

In one embodiment, said pharmaceutical composition is characterized by the concentration in colchicine ranging from 2.5 to 2500 µg per ml of suspension, in particular ranging from 2.5 to 1500 µg per ml of suspension, in particular ranging from 2.5 to 1000 µg per ml of suspension, more particularly from 2.5 to 500 µg per ml of suspension and even more particularly from 2.5 to 250 µg and the anesthetic agent, in particular ropivacaine, is present in a concentration ranging from 0.05 to 120 mg per ml of suspension, in particular from 0.05 to 60 mg per ml of suspension, and more particularly from 0.05 to 20 mg per ml of suspension.

The pharmaceutical composition used in the framework of the present invention may take the form of a sterile and injectable composition, in particular a suspension composition, comprising an effective amount of colchicine.

By "sterile", in the sense of the present invention, is meant an environment capable of guaranteeing to the considered compounds in a composition according to the invention safety requirements for the administration routes such as above-mentioned, notably into or through a joint. Indeed, for obvious reasons, it is essential that a composition according to the invention be devoid of any contaminant body capable of initiating an undesirable side reaction at the host site.

The pharmaceutical composition used in the framework of the present invention may be prepared with the formulation under the form of a powder comprising microparticles as described above. According to one embodiment, said pharmaceutical composition is a sterile and injectable composition for controlled release of colchicine and immediate and/or controlled release of an anesthetic agent, suitable for an intra-articular injection.

By its injectable character, a composition according to the invention necessarily comprises a physiologically acceptable medium, also called "aqueous injection vehicle".

A "physiologically acceptable medium" means a medium devoid of toxicity and compatible with the injection and/or the application of the composition such as considered in the present invention.

The present invention more particularly relates to the pharmaceutical composition as defined above, wherein it is obtained by mixing a formulation under the form of a powder as describe above, with an aqueous injection vehicle, wherein the pharmaceutical composition optionally comprises an excipient selected from a group consisting of a tonicity-enhancing agent, a wetting-agent, a viscosity-enhancing agent, a density-enhancing agent or a mixture thereof, and wherein said excipient may be present in the aqueous injection vehicle or in the powder.

The composition may comprise a solvent or a mixture of physiologically acceptable solvents.

The composition may comprise a physiologically acceptable aqueous medium.

As an aqueous medium suitable for the invention, may be for example mentioned water.

As isotonic agents suitable for the preparation of a composition according to the invention, it may be mentioned sugars and sodium chloride.

The aqueous injection vehicle may in particular comprise a tonicity-enhancing agent, a wetting-agent, a viscosity-enhancing agent or a mixture thereof.

Among the tonicity-enhancing agent, the following may be cited: dextrose, mannitol, sorbitol, sucrose, glycerin, sodium chloride, potassium chloride, cyclodextrin and maltodextrin.

Among the wetting agents, the following may be cited: poly(oxyethylene) sorbitan fatty acid esters, such as commercially available under the trade name TWEEN, sorbitan fatty acid esters, such as commercially available under the trade name SPAN, poloxamer and lecithins.

Among the viscosity-enhancing agents, the following may be cited: sodium carboxymethyl cellulose (CMC), a glycosaminoglycan such as hyaluronic acid, dextran, collagen, poly(vinylpyrrolidone), poly(ethyleneglycol), gelatin, hydroxyethyl cellulose (HEC), methyl cellulose (MC), alginate, gum acacia, starch.

According to a particular embodiment, said pharmaceutical composition has a viscosity of from 5 to 1000 mPa.s, in particular from 5 to 500 mPa.s, in particular from 5 to 100 mPa.s and more particularly from 5 to 50 mPa.s at a shear rate of 10 s⁻¹.

### Kit

Herein is further provided a kit or article of manufacture comprising, in separate compartments (i) an aqueous injection vehicle, optionally comprising an immediate release form of an anesthetic agent, identical or different from the anesthetic agent present in (ii) as defined herein after and (ii) a controlled release dosage form comprising colchicine, in particular as defined above or powder as defined above and an anesthetic agent, in particular as defined above, wherein the kit or article of manufacture optionally comprises an excipient selected from a group consisting of a tonicity-enhancing agent, a wetting-agent, a viscosity-enhancing agent, a density-enhancing agent or a mixture thereof, for preparing a pharmaceutical composition suitable for an intra-articular injection.

Herein is further provided a kit or article of manufacture comprising, in separate compartments (i) an aqueous injection vehicle, optionally comprising an immediate release form of an anesthetic agent, identical or different from the anesthetic agent present in the powder as defined herein after and (ii) a pharmaceutical composition under the form of a powder as defined above, wherein the pharmaceutical composition optionally comprises an excipient selected from a group consisting of a tonicity-enhancing agent, a wetting-agent, a viscosity-enhancing agent, a density-enhancing agent or a mixture thereof may be present in the aqueous injection vehicle or in the powder, for preparing a pharmaceutical composition suitable for an intra-articular injection.

In one embodiment, the kit may be under the form of two separated vials.

In another embodiment, the kit or article of manufacture may be under the form of a vial and a prefilled syringe or of a medical device or is under the form of two separated vials. In said embodiment, the two compartments or dual chamber may allow the mixture of the aqueous injection vehicle and the powder as described herein after by all means known to the man skilled in the art. Still in said embodiment, said means may take the form of a pierceable membrane or destructible diaphragm, for example through a pressure the user may exert.

In these embodiments, the kit or article of manufacture may additionally contain a label instructing the user to introduce the obtained pharmaceutical composition into a subject's joint.

According to one embodiment, said pharmaceutical composition may further comprise an immediate release dosage form containing colchicine. The presence of such immediate release dosage form aims at achieving a quick articular colchicine concentration so as to relieve pain quickly. In one embodiment, the maximal weight ratio of immediate release colchicine relative to the total amount of colchicine is 25%.

In certain embodiment of the invention, the weight ratio of immediate release colchicine relative to the total amount of colchicine may be between 0 and 5%, 0 and 10%, 0 and 15% or 0 and 20%.

In some embodiments, the length of release of the immediate release form is between 0 and 6 hours. In some embodiment, the length of release of the immediate release form is between 0 and 2 hours.

The immediate release dosage form may take the form of immediate fraction that are mixed with the controlled release dosage forms, in particular microparticles as described above or be present in a continuous phase to which the dosage forms, in particular microparticles, are mixed when preparing the pharmaceutical composition according to the present invention. Said continuous phase may of course comprise any further suitable pharmaceutically acceptable excipient than the ones already present within the microparticles.

For the comfort of the patient and for safety reasons as explained above, it is advantageous to look for a pharmaceutical composition, suitable for intra articular injection into a joint, requiring only one injection able to relieve the patient thanks to this injection without needing multiple injections.

### Additional active ingredients

Depending upon the particular condition, or disease, to be treated, additional therapeutic agents, which are normally administered to treat that condition, may be administered in combination with colchicine and the anesthetic agent.

As used herein, the term "combination", "combined," and related terms refers to the simultaneous or sequential administration of said additional active ingredient with colchicine and the anesthetic agent. For example, a combination may be administered with an additional active ingredient simultaneously or sequentially in separate unit dosage forms or together in a single unit dosage form.

According to one embodiment, hyaluronic acid may be injected into the painful joint, and for example be present in pharmaceutical composition according to the present invention. The use of hyaluronic acid in the treatment of arthritis, such as osteoarthritis, is well known, as viscosupplementation. Therefore, according to one embodiment, the present invention provides a pharmaceutical composition comprising colchicine and an anesthetic agent according to the present invention further comprising hyaluronic acid.

In some embodiments, the present invention provides a pharmaceutical composition comprising colchicine and an anesthetic agent according to the present invention further comprising at least an additional therapeutic agent. Suitable additional active ingredients are described in further detail below.

According to one embodiment, the following active ingredients may be combined, in particular any anti-inflammatory active ingredient:
- (i) corticosteroid, such as prednisone, prednisolone, methylprednisolone, betamethasone, dexamethasone, triamcinolone acetonide, triamcinolone hexacetonide, budenoside, mometasone, ciclesonide, fluticasone, hydrocortisone, and pharmaceutically acceptable salts thereof, especially at low dose,
- (ii) an NSAID, such as aspirin, diclofenac, aceclofenac, sulindac, ketorolac, ibuprofen, ketoprofen, naproxen, oxaprozine, flubiprofen, indomethacin, proglumetacin, tiaprofenic acid, meloxicam, piroxicam, tenoxicam, etodolac and celecoxib, etoricoxib, parecoxib, rofecoxib, valdecoxib, and pharmaceutically acceptable salts thereof,
- (iii) an antiIL-1β agents such as anakinra, canakinumab, rilonacept, and pharmaceutically acceptable salts thereof,
- (iv) Anti-IL-6 agents such as tocilizumab, siltuximab, sarilumab, and pharmaceutically acceptable salts thereof,
- (v) an anti-TNFα agent, such as adalimumab, etanercept, infliximab, certolizumab, golimumab, and pharmaceutically acceptable salts thereof,
- (vi) an anti-angiogenic agents such as bevacizumab, and pharmaceutically acceptable salts thereof,
- (vii) an anti NGF (Nerve Growth Factors) agent, such as fasinumab, tanezumab, fulranumab, ABT-110, and pharmaceutically acceptable salts thereof,
- (viii) an opioid, such as fentanyl, morphine, buprenorphine, hydromorphone, hydrocodone, oxycodone, meperidine, and pharmaceutically acceptable salts thereof,
- (ix) an inhibitor of class I glucose transporters (e.g. GLUT-1/GLUT-3), such as cytochalasin B, WZB-117, STF-31, BAY-876,
- (x) mixtures thereof.

Said additional active ingredient may be formulated under immediate and/or controlled release dosage forms.

### Administration of the composition

A pharmaceutical composition used in the framework of the present invention can be injected using any of the known methods in the art.

Particularly, said pharmaceutical composition may be administered by means of an injection device suitable for intra-articular injection such as a syringe equipped with a needle 19-29G, preferentially 22-29G, more preferentially 25-29G.

Throughout the description, including the claims, the expression "comprising a" should be understood as being synonymous with "comprising at least one" unless specifically stated otherwise.

The expressions "between... and ..." and "ranging from ... to ..." should be understood to mean that the limits are inclusive, unless specified otherwise.

The following examples and figures are presented by way of non-limiting illustration of the invention.

### EXAMPLES

### Analytical methods

- Particle size was determined using a Malvern Mastersizer MS3000 laser diffraction particle size analyzer. A 1 mg/ml microparticles in deionized water preparation was introduced in the apparatus and the measurement run 3 times. The results shown in the examples are the mean of the three measures.
- Colchicine Microparticle drug load was determined by dissolving 15 mg of particles with 1,25 mL acetonitrile. After complete dissolution, 11,25 mL of methanol was added. Then 12.5 ml of deionized water were introduced and the mixture thoroughly mixed using vortex agitation. The medium was then centrifuged for 30 min at 4000 rpm and the supernatant filtrated through a 0.45 µm PTFE filter (Acrodisc wwPTFE),. A 2 ml aliquot of the filtrated supernatant was then analyzed **by HPLC** to determine the microparticle drug load. **HPLC** was conducted on an Agilent HP 1200 system, using a silica-based reversed-phase C8 column [GL Sciences, Inertsil C8-3, 4.6 × 250 mm (5µm)] set at 25°C. The mobile phase consisted of 55% methanol and 45% of a 6.8 g/L KH2PO4 solution previously adjusted to pH 5.5 with diluted phosphoric acid. The flow rate was set at 1 mL/min, the injection volume was of 20 µL and the UV detection was set at 254 nm.
- **Ropivacaine Microparticle drug load** was determined by dissolving 15 mg of particles with 1,25 mL acetonitrile. After complete dissolution, 11,25 mL of methanol was added. Then 12.5 ml of deionized water were introduced and the mixture thoroughly mixed using vortex. The medium was then centrifuged for 30 min at 4000 rpm and the supernatant filtrated through a 0.45 µm PTFE filter (Acrodisc wwPTFE. A 2 ml aliquot of the filtered supernatant was then analyzed **by HPLC** to determine the microparticle drug load. **HPLC** was conducted on an Agilent HP 1200 system, using a silica-based reversed-phase C8 column [GL Sciences, Inertsil C8-3, 4.6 × 250 mm (5µm)] set at 25°C. The mobile phase consisted of 40% acetonitrile and 60% of a 6.8 g/L KH2PO4 solution previously adjusted to pH 5.5 with diluted phosphoric acid. The flow rate was set at 1 mL/min, the injection volume was of 20 µL and the UV detection was set at 215 nm.
- For the ***in vitro* release tests from Colchicine microparticles,** aliquots of 20 mg microparticles were suspended in 50 mL of phosphate buffered saline (10 mM, pH 7.4) maintained at 37°C in a horizontal shaking (incubator model 3033 commercialized by GFL). At different intervals, 3 mL of the media were removed and centrifuged at 4000 rpm for 3 minutes. The supernatant was analyzed by UV (FastTrack - Mettler Toledo) at 350 nm. Right after UV analysis, the sample was put back in the dissolution flask.
- For the ***in vitro* release tests from Ropivacaine microparticles,** aliquots of 20 mg microparticles were suspended in 20 mL of phosphate buffered saline (10 mM, pH 7.4) maintained at 37°C in a horizontal shaking (incubator model 3033 commercialized by GFL). At different intervals, 1 mL of the medium was removed and filtrated through a 0.45 µm PTFE filter (Acrodisc wwPTFE) while the withdrawn volume from the vessel, was replaced by fresh medium. The filtrated sample was then analyzed by HPLC as described above.

### Example 1: Sustained-release Colchicine microparticles and pharmaceutical composition comprising it

Colchicine microparticles were prepared under laminar flow and using autoclaved and sterilized containers as follows: A 1 wt% polyvinyl alcohol (PVA, Mowiol 4-88, Sigma-Aldrich) stock solution was prepared by heating 2970 g sterile water (Gibco15230-162) to 70°C and dispersing therein 30 g of PVA under magnetic stirring until full solubilization. The solution was then allowed to cool down prior to further use.

In a separate autoclaved container, 4.03 g of colchicine (INDENA, Italy) were solubilized in 10.75 mL methylene chloride (Merck) under magnetic stirring. Then, 6.02 g of PLGA 50:50 Resomer 503H (Evonik Industries AG, Essen, Germany) were introduced in the mixture and completely dissolved under magnetic stirring to form a polymer-drug solution.

28.6 g of the previously prepared PVA 1 wt% stock solution were then added to the drug-polymer solution under high shear (11 000 RPM) for a duration of 60 seconds, using an IKA T25 Ultra-Turrax rotor stator mixer equipped with a S25N 10G head.

This emulsion was then slowly poured into a hardening bath containing 2860 g of the PVA 1 wt% stock solution previously prepared.

Agitation was maintained using a double propeller stirrer with a stirring speed set at 300 RPM for 3 hours until methylene chloride is evaporated.

The formed microparticles were centrifuged at 4000 RPM for 3 minutes using a swinging bucket GT2R centrifuge apparatus to remove the main portion of the water phase and get a concentrated particle slurry prior vacuum filtration over a 12-25 µm Whatman cellulose filter and washing with 150 ml water.

The washed microparticles were then vacuum dried at 0.08 mBar and 15°C shelf temperature for 17 hours.

The collected microparticles were then stored at 5°C.

Analytical tests, including the *in vitro* profile, were performed as in Example 1, and are shown in Table 1.

**Table 1. In vitro release profile for Colchicine PLGA microparticles**

| Colchicine loading content (wt %) | *In vitro* release in 10 mM PBS, pH 7.4 n=3 (%) |
|---|---|
| 22 | 0.08 day: 7.9 |
| | 1 day: 29.1 |
| | 2 days: 39.9 |
| | 3 days: 48.8 |
| | 4 days: 55.8 |
| | 7 days: 72.2 |

In this example, colchicine loaded PLGA microparticles were prepared with a drug loading content of 22 wt %.

An aqueous injection vehicle was prepared under laminar flow using pyrogen-free excipients and consisting of 1.4% of low viscosity sodium carboxymethyl cellulose (Aqualon CMC 7LF PH BET, Ashland), 0.1% Polysorbate 20 (Emprove Essential from Merck), 0.13% disodium hydrogen phosphate dihydrate (Roth), 0.1% citric acid (Roth) and 0.65% sodium chloride (Roth). Final pH of the solution was adjusted to 6.9 using concentrated sodium hydroxide solution (Roth). The vehicle was then autoclaved at 121°C for 20 minutes (Systec 3150EL) and 55 mL aliquots of the solution transferred aseptically into 100 mL vials under laminar air flow.

15 min prior to *in vivo* injection as described in following example 10, a 10 mg aliquot of microparticles as prepared according to this example was dispersed in 55 mL of injection vehicle using sonication for 30 seconds until homogeneous dispersion was obtained. The colchicine loaded PLGA microparticles suspension thus prepared, yielded a colchicine concentration of 0.04 mg/ml or 40 µg/ml.

### Example 2: Sustained-release Colchicine microparticles

Colchicine microparticles were prepared as follows: A 1 wt% polyvinyl alcohol (PVA, Mowiol 4-88, Sigma-Aldrich) stock solution was prepared by heating 8712 g deionized water to 70°C and dispersing therein 88 g of PVA under magnetic stirring until full solubilization. The solution was then allowed to cool down prior to further use.

In a separate container, 1.5 g of colchicine (INDENA, Italy) were solubilized in 32.25 mL methylene chloride (Merck) under magnetic stirring. Then, 8.55 g of PLGA 50:50 Resomer 503H and 0.45 g of PLGA 50:50 Resomer 502H (both from Evonik Industries AG, Essen, Germany) were introduced in the mixture and completely dissolved under magnetic stirring to form a polymer-drug solution.

85.8 g of the previously prepared PVA 1 wt% stock solution were then added to the drug-polymer solution under high shear (9 500 RPM). Emulsification was performed over a duration of 10 minutes using an IKA T25 Ultra-Turrax rotor stator mixer equipped with a S25N 10G head.

This emulsion was then slowly poured into a hardening bath containing 7000 g of the PVA 1 wt% stock solution previously prepared.

Agitation was maintained using a double propeller stirrer with a stirring speed set at 300 RPM for 3 hours until methylene chloride is evaporated.

The formed microparticles were centrifuged at 4000 RPM for 3 minutes using a swinging bucket GT2R centrifuge apparatus to remove the main portion of the water phase and get a concentrated particle slurry. The slurry was then redispersed in about 20 ml water and the redispersed particles sieved over 40µm, then 10µm using stainless steel Endecotts sieves.

The collected particles were then vacuum filtered over a 12-25 µm Whatman cellulose filter and washed with 150 ml water.

The washed microparticles were then vacuum dried at 2 mBar and 15°C shelf temperature for 17 hours.

The collected microparticles were then stored at 5°C.

Analytical tests, including the *in vitro* profile, were performed as in Example 1, and are shown in Table 2.

**Table 2. In vitro release profile for Colchicine PLGA microparticles**

| Colchicine loading content (wt %) | *In vitro* release in 10 mM PBS, pH 7,4 n=2 (%) | Particle size (Dv, µm) |
|---|---|---|
| 4.3 | 0.04 day: 8.3 | D10 = 11 µm |
| | 0.08 day: 15.8 | D90 = 39 µm |
| | 1 day: 27.3 | |
| | 2 days: 44.0 | Mean Particle Size = 24 µm |
| | 7 days: 87.1 | |

In this example, colchicine loaded PLGA microparticles were prepared with a drug loading content of 4.3 wt % with a mean particle size of 24 µm.

### Example 3: Sustained-release Colchicine microparticles

Colchicine microparticles were prepared under laminar flow and using autoclaved and sterilized containers as follows: A 1 wt% polyvinyl alcohol (PVA, Mowiol 4-88, Sigma-Aldrich) stock solution was prepared by heating 2970 g of sterile water (Gibco15230-162) to 70°C and dispersing therein 30 g of PVA under magnetic stirring until full solubilization. The solution was then allowed to cool down prior to further use.

In a separate autoclaved container, 0.5 g of colchicine (INDENA, Italy) were solubilized in 10.75 mL methylene chloride (Merck) under magnetic stirring. Then, 2.85 g of PLGA 50:50 Resomer 503H and 0.15 g of PLGA 50:50 Resomer 502H (both from Evonik Industries AG, Essen, Germany) were introduced in the mixture and completely dissolved under magnetic stirring to form a polymer-drug solution.

28.6 g of the previously prepared PVA 1 wt% stock solution were then added to the drug-polymer solution under high shear (9 500 RPM). Emulsification was performed over a duration of 10 minutes using an IKA T25 Ultra-Turrax rotor stator mixer equipped with a S25N 10G head.

This emulsion was then slowly poured into a hardening bath containing 2860 g of the PVA 1 wt% stock solution previously prepared.

Agitation was maintained using a double propeller stirrer with a stirring speed set at 300 RPM for 3 hours until methylene chloride is evaporated.

The formed microparticles were centrifuged at 4000 RPM for 3 minutes using a swinging bucket GT2R centrifuge apparatus to remove the main portion of the water phase and get a concentrated particle slurry. The slurry was then redispersed in about 20 ml water and the redispersed particles sieved over 40µm, then 10µm using stainless steel Endecotts sieves.

The collected particles were then vacuum filtered over a 12-25 µm Whatman cellulose filter and washed with 150 ml sterile water.

The washed microparticles were then vacuum dried at 2 mBar and 15°C shelf temperature for 17 hours.

The collected microparticles were then stored at 5°C.

Analytical tests, including the *in vitro* profile, were performed as in Example 1, and are shown in Table 3.

**Table 3. In vitro release profile for Colchicine PLGA microparticles**

| Colchicine loading content (wt %) | *In vitro* release in 10 mM PBS, pH 7,4 n=2 (%) | Particle size (Dv, µm) |
|---|---|---|
| 5.1 | 0.04 day: 9.7 | D10 = 11 µm |
| | 0.08 day: 15.7 | D90 = 37 µm |
| | 1 day: 23.9 | |
| | 2 days: 40.8 | Mean Particle Size = 21 µm |

In this example, colchicine loaded PLGA microparticles were prepared with a drug loading content of 5.1 wt % with a mean particle size of 21 µm.

### Example 4: Sustained release Ropivacaine microparticles

Ropivacaine microparticles were prepared as follows: 4.01 g of Ropivacaine HCl (Benechim, Belgium) were dispersed in 100 ml deionized water until complete solubilization. pH was then adjusted to 9.9 and Ropivacaine base thus obtained under the form of a precipitate. The solid was filtered (Büchner; 11µm) and vacuum dried at room temperature for 17 hours. About 2.6 g of Ropivacaine base were collected.

Separately, a 1 wt% polyvinyl alcohol (PVA, Mowiol 4-88, Sigma-Aldrich) stock solution was prepared by heating 3069 g of deionized water to 70°C and dispersing therein 31 g of PVA under magnetic stirring until full solubilization. The solution was then allowed to cool down prior to further use.

In a separate container, 0.5 g of ropivacaine base previously collected were solubilized in 10.75 mL methylene chloride (Merck) under magnetic stirring. Then, 3 g of PLGA 50:50 Resomer 502H (Evonik Industries AG, Essen, Germany) were introduced in the mixture and completely dissolved under magnetic stirring to form a polymer-drug solution.

26.3 g of the previously prepared PVA 1 wt% stock solution were then slowly added to the drug-polymer solution under high shear (9 500 RPM). Emulsification was performed over a duration of 10 minutes using an IKA T25 Ultra-Turrax rotor stator mixer equipped with a S25N 10G head.

This emulsion was then slowly poured into a hardening bath containing 2989 g of the PVA 1 wt% stock solution previously prepared.

Agitation was maintained using a double propeller stirrer with a stirring speed set at 300 RPM for 3 hours until methylene chloride is evaporated.

The formed microparticles were centrifuged at 4000 RPM for 3 minutes using a swinging bucket GT2R centrifuge apparatus to remove the main portion of the water phase and get a concentrated particle slurry. The slurry was then redispersed in about 20 ml water and the redispersed particles sieved over 40 µm stainless steel Endecotts sieves.

The collected particles were then vacuum filtered over a 12-25 µm Whatman cellulose filter and washed with 150 ml water.

The washed microparticles were then vacuum dried at 2 mBar and 15°C shelf temperature for 17 hours.

The collected microparticles were then stored at 5°C.

Analytical tests, including the *in vitro* profile, were performed as in Example 1, and are shown in Table 4.

**Table 4. In vitro release profile for Ropivacaine PLGA microparticles**

| Ropivacaine loading content (wt %) | *In vitro* release in 10 mM PBS, pH 7,4 n=2 (%) | Particle size (Dv, µm) |
|---|---|---|
| 5.7 | 0.5 hours: 8.6 | |
| | 1 hour: 16.8 | D10 = 7 µm |
| | 2 hours: 37.8 | D90 = 25 µm |
| | 4 hours: 54.4 | |
| | 9 hours: 70.0 | Mean Particle Size = 13 µm |
| | 14 hours: 76.5 | |
| | 17 hours: 82.5 | |
| | 24 hours: 96.9 | |

In this example, ropivacaine-loaded PLGA microparticles were prepared with a drug loading content of 5.7 wt % with a mean particle size of 13 µm.

### Example 5: Ropivacaine Hydrogel

A 20 mg/ml Ropivacaine stock solution was prepared by solubilizing 201 mg Ropivacaine HCl (Benechim, Belgium) in 10 ml deionized water. When completely solubilized, 150 mg of Sodium hyaluronate 1,500kDa (H-1500 from Tebubio) were dispersed in the Ropivacaine stock solution under magnetic stirring for about 4 hours until complete solubilization. The mixture was then covered and left at room temperature overnight.

The next day, 1 ml aliquots of the prepared ropivacaine hydrogel were put into dialysis bags (12-14 KDa) and immersed in 50ml Schott flasks filled with 25 ml pPhosphate buffered saline 10 mM. The containers were closed and left under magnetic agitation.

At determined intervals, 500µL of the medium were pulled out and the withdrawn volume replaced with fresh PBS 10mM medium. The quantification of released ropivacaine was assessed by HPLC as described in the analytical methods section and the results shown in Table 5

**Table 5. In vitro release profile for Ropivacaine hydrogel**

| *Time* | *In vitro* release in 10 mM PBS, pH 7.4 n=2 (%) |
|---|---|
| 0.5 hours | 39.1 |
| 1 hour | 56.2 |
| 1.5 hours | 75.5 |
| 2 hours | 83.8 |
| 6 hours | 86.4 |
| 24 hours | 99.5 |

### Example 6: Pharmaceutical composition

An aqueous injection vehicle was prepared under laminar flow using water for injection and pyrogen-free excipients. The vehicle consisted of 1.4% of low viscosity sodium carboxymethyl cellulose (Aqualon CMC 7LF PH BET, Ashland), 0.1% Polysorbate 20 (Emprove Essential from Merck), 0.13% disodium hydrogen phosphate dihydrate (Roth), 0.1% citric acid (Roth) and 0.65% sodium chloride (Roth). Final pH of the solution was adjusted to 7 using concentrated sodium hydroxide solution (Roth).

The vehicle was then autoclaved at 121°C for 20 minutes (Systec 3150EL) and finally stored at 4°C for later use.

The pharmaceutical composition was prepared by weighing in a 20 ml previously autoclaved container, 10 mg of colchicine microparticles prepared according to example 3, 860 mg of ropivacaine microparticles prepared according to example 4 and 51 mg of ropivacaine HCl.

12.85 ml of the aqueous injection vehicle were introduced in the vial and homogenization performed until complete dispersion of the particles. The suspension thus prepared, yielded a colchicine concentration of 40 µg/ml, a ropivacaine concentration conveyed under the form of microparticles of 3.8 mg/ml and a ropivacaine concentration conveyed under the form of immediate release of 4 g/ml. 50 µL of this suspension were then injected in inflamed rat ankle joints.

## Claims

1. A pharmaceutical composition suitable for an intra-articular injection comprising an anesthetic agent and a controlled release dosage form comprising colchicine.

2. The pharmaceutical composition according to claim 1, wherein the controlled release dosage form comprising colchicine is under the form of an in-situ forming depot, a hydrogel or microparticles, in particular microparticles comprising a polymeric matrix or multivesicular liposomes.

3. The pharmaceutical composition according to claim 2, wherein the controlled release dosage form comprising colchicine is under the form of microparticles, in particular microparticles comprising a polymeric matrix, and wherein said microparticles comprising colchicine have a mean particle size equal or greater than 10 µm, and in particular have a mean particle size lower than 100 µm, more particularly lower than 80 µm, and even more particularly lower than 50 µm, for example between 10 and 50 µm or between 10 and 40 µm.

4. The pharmaceutical composition according to claim 3, wherein the microparticles are microparticles comprising colchicine and a polymeric matrix, wherein the polymeric matrix comprises at least a poly(lactic-co-glycolic acid) copolymer, at least poly(caprolactone) or at least a mixture of poly(lactic-co-glycolic acid) copolymer and poly(caprolactone).

5. The pharmaceutical composition according to claim 3 or 4, wherein the percentage weight ratio between the colchicine and the total weight of the microparticles or drug loading content ranges from 0.1 to 35% by weight, in particular from 0.5 to 30% by weight, more particularly from 1 to 25% by weight, and even more particularly from 1 to 10% by weight.

6. The pharmaceutical composition according to anyone of claims 3 to 5, wherein the polymeric matrix further comprises one or more additional polymer(s) or copolymer(s) selected from poly(lactide) distinct from poly(lactic-co-glycolic acid) copolymer, poly(glycolide) distinct from poly(lactic-co-glycolic acid) copolymer, poly(lactide-co-caprolactone), poly(ethylene glycol), poly(ethylene oxide), PLGA-b-PEO-b-PLGA, PLGA-b-PEO, polyhydroxyalkanoates, poly(hydroxybutyrate), poly(trimethylene carbonate), poly(dioxanone), poly(valerolactone), poly(alpha- hydroxy acids), poly(lactones), poly(amino acids), polyanhydrides, poly(orthoester), poly(acetals), polyurethanes, polythioesters, polyphosphoesters, poly(ester-co-amide), poly(vinyl alcohol), PVA-g-PLGA, poly (ether ester) multiblock copolymers, polyvinyl pyrrolidone, poly(methacrylate), PEO-PPO-PEO, gelatin, heparin, chondroitin sulfate; polysaccharides such as alginates, starch, chitosan and dextrans and any combinations thereof, and in particular selected from poly(lactide) and poly(caprolactone).

7. The pharmaceutical composition according to anyone of claims 3 to 6, wherein the polymeric matrix comprises poly(lactic-co-glycolic acid), copolymer poly(caprolactone), or a mixture of poly(lactic-co-glycolic acid) copolymer and poly(caprolactone) in an amount of greater than 70% by weight, with respect to the total weight of the polymeric matrix, in particular greater than 80% by weight, and even more particularly greater than 90% by weight.

8. The pharmaceutical composition according to anyone of claims 1 to 7, wherein the anesthetic agent is selected from lidocaine, ropivacaine, bupivacaine, levobupivacaine, capsaicin, mepivacaine, prilocaine, pharmaceutically acceptable salts thereof and mixtures thereof, in particular from lidocaine, bupivacaine, ropivacaine and mepivacaine, and more particularly ropivacaine.

9. The pharmaceutical composition according to anyone of claims 1 to 8, wherein the anesthetic agent is partially or totally under an immediate release form or is partially or totally under a controlled release form, said controlled release dosage form being distinct from the controlled release dosage form comprising colchicine or totally of partially being comprised in it, such as under the form of microparticles comprising a polymeric matrix, in particular as defined in anyone of claims 3 to 7, under the form of a hydrogel, under the form of multivesicular liposomes or under the form of an in-situ forming depot.

10. A pharmaceutical composition under the form of a powder comprising an anesthetic agent and controlled release dosage form comprising colchicine, wherein said controlled release dosage form comprising colchicine are under the form of microparticles, in particular microparticles comprising a polymeric matrix, more particularly having a mean particle size equal or greater than 10 µm, and even more particularly as defined in any one of claims 3 to 7, said anesthetic agent being in particular as defined in claim 8 or 9.

11. The pharmaceutical composition according to anyone of claims 1 to 9, wherein it is under the form of a sterile and injectable dosage form selected from a solution, a suspension, a solid implant, a semi-solid implant, a powder and an in-situ depot.

12. The pharmaceutical composition according to claim 11, in particular under the form of a sterile and injectable suspension, optionally comprising an excipient selected from a group consisting of a tonicity-enhancing agent, a wetting-agent, a viscosity-enhancing agent, a density-enhancing agent or a mixture thereof, and wherein it is obtained by mixing controlled release dosage form comprising colchicine, in particular microparticles comprising colchicine, more particularly as defined in anyone of claims 3 to 7 with an aqueous injection vehicle and an anesthetic agent, in particular as defined in claim 8 or 9.

13. The pharmaceutical composition according to claim 11 or 12, wherein the colchicine is present in a concentration ranging from 2.5 to 2500 µg per ml of the sterile and injectable dosage form, in particular a suspension, in particular from 2.5 to 1500 µg, in particular from 2.5 to 1000 µg per ml of the sterile and injectable dosage form, in particular a suspension, and more particularly from 2.5 to 500 µg per ml, and even more particularly from 2.5 to 250 µg per ml.

14. The pharmaceutical composition according to anyone of claims 11 to 13, wherein the anesthetic agent, is present in a concentration ranging from 0.05 to 120 mg per ml of the sterile and injectable dosage form, in particular a suspension, in particular from 0.05 to 60 mg per ml, and more particularly from 0.05 to 20 mg per ml.

15. A kit or article of manufacture comprising, in separate compartments (i) an aqueous injection vehicle, optionally comprising an immediate release form of an anesthetic agent, identical or different from the anesthetic agent present in (ii) as defined herein after and (ii) a controlled release dosage form comprising colchicine, in particular as defined in anyone of claims 2 to 7 or powder as defined in claim 10 and an anesthetic agent, in particular as defined in claim 8 or 9, wherein the kit or article of manufacture optionally comprises an excipient selected from a group consisting of a tonicity-enhancing agent, a wetting-agent, a viscosity-enhancing agent, a density-enhancing agent or a mixture thereof, for preparing a pharmaceutical composition suitable for an intra-articular injection.

16. The kit or article of manufacture according to claim 15, wherein it is under the form of a vial and a prefilled syringe or of a medical device or is under the form of two separated vials.

17. A pharmaceutical composition according to anyone of claims 1 to 9 and 11 to 14 or as obtained by mixing the two compartments of a kit according to claim 15 or 16, for use in the treatment of crystal-associated and non-crystal associated acute inflammatory arthritis, in particular a crystal-associated arthropathy or flares or acute forms of tendinitis and capsulitis by intra-articular injection into a joint of said pharmaceutical composition, in particular providing pain relief and inflammation decrease, and more particularly immediately and all over the flare duration, wherein the time required to release 80% by weight of the colchicine is greater than 1 day and may reach 15 days, wherein the colchicine is present in a concentration ranging from 2.5 to 2500 µg per ml of the sterile and injectable dosage form, in particular a suspension, wherein the anesthetic agent is present in a concentration ranging from 0.05 to 120 mg per ml of the sterile and injectable dosage form, in particular a suspension, wherein 80% of the anesthetic agent is released in maximum 3 days and wherein the pharmaceutical composition has a volume ranging from 0.1 ml to 5 ml.

18. The pharmaceutical composition for use according to claim 17, wherein the crystal-associated and non-crystal associated acute inflammatory arthritis is selected from non-crystal associated acute arthritis, such as Rheumatoid arthritis, Ankylosing spondylitis, Juvenile arthritis, Psoriatic arthritis, Lupus and crystal-associated arthropathy attacks or flares or acute forms of tendinitis and capsulitis, and more particularly selected from gout, chondrocalcinosis, calcifying tendinitis and Milwaukee shoulder syndrome.

19. The pharmaceutical composition for use according to claim 17 or 18, wherein the time required to release 80% by weight of the colchicine is greater than 1 day, for example 1.5 day, and may reach 10 days, is greater than 1 day, for example 1.5 day, and may reach 7 days, is greater than 1 day, for example 1.5 day, and may reach 5 days, and for example is greater than 1 day, for example 1.5 day, 2 days, 3 days, 4 days, 5 days, 6 days, 7 days, 8 days, 9 days or 10 days, more particularly is greater than 1 day, for example 1.5 day, and may reach 7 days, or is comprised between 2 days and 15 days, between 2 days and 10 days and between 2 days and 7 days and wherein the time required to release 80% by weight of the anesthetic agent is maximum 3 days, for example maximum 2 days and in particular maximum 1 day.

20. The pharmaceutical composition for use according to anyone of claims 17 to 19, wherein the pharmaceutical composition is further **characterized in that** it is effective to maintain systemic concentration of colchicine 12 hours after the intra-articular injection lower than 5 ng/ml, particularly lower than 2 ng/ml, and more particularly lower than 1 ng/ml and to maintain synovial concentration of colchicine greater than 1 ng/ml, in particular comprised between 1 and 500 ng/ml, more particularly comprised between 1 and 250 ng/ml, more particularly comprised between 1 and 200 ng/ml, for example between 1 and 100 ng/ml, and even more particularly between 1 and 50 ng/ml.

21. The pharmaceutical composition for use according to anyone of claims 17 to 20, wherein the pharmaceutical composition is further **characterized in that** it is effective to maintain synovial concentration of anesthetic agent, in particular ropivacaine, comprised between 0.05 and 50 mg/ml, in particular between 0.05 and 25 mg/ml, and more particularly between 0.05 to 10 mg/ml, for example between 0.05 and 3 mg/ml, said synovial concentration being able to last 1 hour, 4 hours, 8 hours, 1 day, 1.5 days or 2 days or 3 days.

22. The pharmaceutical composition for use according to anyone of claims 17 to 21, wherein it is for use in the treatment of crystal-associated and non-crystal associated acute inflammatory arthritis, in particular a crystal-associated arthropathy or flares or acute forms of tendinitis and capsulitis in a patient have renal and/or hepatic impairment or in a patient being simultaneously, separately or sequentially and non-exclusively treated by at least one active ingredient selected from: atazanavir, clarithromycin, darunavir, ritonavir, indinavir, itraconazole, ketoconazole, lopinavir, nefazodone, nelfinavir, saquinavir, telithromycin, tipranavir, amprenavir, aprepitant, diltiazem, erythromycin, fluconazole, fosamprenavir, verapamil, cyclosporine, ranolazine, macrolides and statins.
